# EUROPEAN PATENT APPLICATION

(11) **EP 4 431 639 A1**
(43) Date of publication of application: **18.09.2024**
(21) Application number: 22891700.1
(22) Date of filing: 08.10.2022
(51) Int. Cl.: C25B 1/04, C25B 9/00, A61L 2/10, A61L 9/20

(54) **HYDROGEN GENERATOR HAVING SELF-DISINFECTION FUNCTION**

(30) Priority: 09.11.2021 CN 202111316871
(71) Applicant: Lin, Hsin-Yung, Taoyuan, Taiwan 33341 (TW)
(72) Inventor: Lin, Hsin-Yung, Taoyuan, Taiwan 33341 (TW)
(74) Representative: Wang, Bo
(86) International application number: PCT/CN2022/123747
(87) International publication number: WO 2023/082899

(57) **Abstract**

A hydrogen generator with self-sterilization function comprises an electrolysis module, a hydrogen water cup, an integrated channel device and an automatic diversion device. The electrolysis module is configured to electrolyze electrolytic water to generate the gas comprising hydrogen. The hydrogen water cup is configured to accommodate liquid and inputting the gas comprising hydrogen into the liquid to generate the liquid comprising hydrogen. The integrated channel device is stacked above the electrolysis module and comprises a gas input channel, a gas output channel, and a gas flow channel. The automatic diversion device is configured for selectively connecting the gas input channel, the hydrogen water cup and the gas output channel or selectively connecting the gas input channel, the gas flow channel and the gas output channel. Wherein, the pH value of the electrolytic water in the electrolysis module is in a range between 12-14.

## Description

### BACKGROUND OF THE INVENTION

### 1. Field of the invention

The present invention relates to a hydrogen generator, and more particularly, a hydrogen generator with self-sterilization function to provide clean gas comprising hydrogen and reduce its maintenance costs.

### 2. Description of the prior art

People always pay much attention to life extension, so many medical technologies are developed to fight diseases. However, the development of medical technology has been concentrating on preventive medical methods, such as healthy food research, genetic disease screening and disease prevention, and even has been actively taking initiatives to prevent possible future diseases.

Researches show that the body's unstable oxygen gas (O+), also known as free radicals (harmful free radicals), is caused by various reasons such as disease, diet, environment or living habits. Free radical is an atom, molecule or ion with a single unpaired electron. Free radical attacks human cell membranes, cells and tissues to steal electrons from other atoms, so as to cause a chain peroxidation reaction in the body. The chain peroxidation reaction will cause degenerative symptoms within the body, such as fragile blood vessels, Dementia, weakened immune system, cataracts, degenerative arthritis, sagging skin and general aging. Many researches show that hydrogen-rich water (hydrogen gas water or hydrogen water) has small molecular groups, so it can easily enter cell channels to be absorbed and then participate in the body's metabolism to promote cell detoxification. Therefore, drinking hydrogen-rich water can reduce the number of free radicals in the human body and restore the acidic body to a healthy alkaline body, thereby also achieving the elimination of chronic diseases and the positive effects of beauty and health care.

In the prior art, hydrogen generators that can produce water comprising hydrogen at the same time mostly use electrolysis modules to generate gas comprising hydrogen and then inject the gas comprising hydrogen into the drinking water, and output some gas comprising hydrogen which has not been dissolved in the drinking water for user inhaling. However, when the gas comprising hydrogen is injected into the drinking water, the hydrogen generator will generate low-frequency sounds. If a user uses the hydrogen generator to inhale the gas comprising hydrogen while sleeping, the low-frequency sounds will easily affect the user's sleep quality and health. Therefore, it is necessary to solve the connection problem between the hydrogen water cup and the electrolysis module.

In addition, the hydrogen generator usually produces the water comprising hydrogen by directly injecting the gas comprising hydrogen into the water through a tube. However, the bubbles of the gas comprising hydrogen injected into the water of the method of the prior art is not fine enough which will lead the contact area between the gas comprising hydrogen and water is too small to dissolve smoothly in water. According to documents published by the International Hydrogen Molecular Standards Association (IHSA) in 2017, the documents showed that the concentration of hydrogen in the water comprising hydrogen had to be greater than 0.5ppm of the mass concentration to produce biological effects. However, the maximum physical limit of the water comprising hydrogen is 1.6ppm under standard conditions, that is, under one atmosphere and at 20°C. Therefore, how to make the water comprising hydrogen that is greater than 0.5ppm and can reach 1.6ppm is another topic to be solved urgently.

Moreover, there is no self-sterilization or self-disinfection function in the hydrogen generator of the prior art. After using the hydrogen generator of the prior art for a period of time, it is necessary to disassemble the hydrogen generator to get disinfected or sterilized. If a user does not disassemble the hydrogen generator to clean, the hydrogen generator may generate the gas comprising hydrogen with germs, which will easily lead the user to get sick after inhaling. In addition, it will significantly increase the maintenance cost of the hydrogen generator of the prior art when the user disassemble the entire hydrogen generator for disinfection and sterilization.

### SUMMARY OF THE INVENTION

Therefore, the present invention provides a hydrogen generator with self-sterilization function to solve the problems of the prior art.

In one embodiment of the present invention, the hydrogen generator with self-sterilization function comprises an electrolysis module, a hydrogen water cup, an integrated channel device, and an automatic diversion device. The electrolysis module is configured to electrolyze an electrolytic water to generate a gas comprising hydrogen. The hydrogen water cup is configured to accommodate a liquid and is inputted the gas comprising hydrogen into the liquid to generate a liquid comprising hydrogen. The integrated channel device is stacked above the electrolysis module. The integrated channel device comprises a gas input channel, a gas output channel, and a gas flow channel. Wherein, the gas input channel is configured to receive the gas comprising hydrogen, and the gas output channel is configured to output the gas comprising hydrogen. The automatic diversion device is selectively connected with the gas input channel, the hydrogen water cup and the gas output channel to input the gas comprising hydrogen into the hydrogen water cup and to output the gas comprising hydrogen through the gas output channel. The automatic diversion device is selectively connected with the gas input channel, the gas output channel and the gas flow channel to input the gas comprising hydrogen into the hydrogen water cup and to output the gas comprising hydrogen through the gas output channel; or selectively connected with the gas input channel, the gas flow channel and the gas output channel to output the gas comprising hydrogen through the gas flow channel and the gas output channel. Wherein, the pH value of the electrolytic water in the electrolysis module is in a range between 12-14 to generate a strong alkaline environment for sterilization.

Wherein, the internal temperature of the electrolysis module is in a range between 50°C to 80°C while the electrolysis module performs electrolysis.

Wherein, the pH value of the electrolytic water in the electrolysis module is in a range between 13-13.9.

Wherein, the hydrogen generator further comprises a nebulizer coupled to the gas output channel to receive the gas comprising hydrogen. Wherein, the nebulizer selectively generates an atomized gas to be mixed with the gas comprising hydrogen to form a healthy gas.

Wherein, the nebulizer generates the atomized gas when the gas comprising hydrogen is inputted into the hydrogen water cup and then outputted from the gas output channel; the nebulizer stops generating the atomized gas when the gas comprising hydrogen is outputted by the gas output channel through the gas flow channel.

Wherein, the hydrogen generator further comprises a frame for the hydrogen water cup to be embedded in to couple the hydrogen water cup to the integrated channel device. Wherein the electrolysis module stops operating when the hydrogen water cup is detached from the frame and is not coupled to the integrated channel device.

Wherein, the hydrogen generator further comprises a condensation filter device coupled to the integrated channel device to condense and filter the gas comprising hydrogen. Wherein, the integrated channel device comprises a lower cover. The lower cover is an integrally formed structure and directly coupled to the automatic diversion device and the condensation filter device. The lower cover has a space configured to accommodate the condensation filter device and a movable flip-up structure, configured to movably embed the condensation filter device into the integrated channel device. Wherein, the gas comprising hydrogen is transferred among the hydrogen water cup, the automatic diversion device and the condensation filter device by the integrated channel device.

Wherein, the hydrogen generator further comprises a water tank, a humidifying cup and a filter. The water tank is stacked below the integrated channel device and coupled to the electrolysis module. The water tank is configured to accommodate the electrolytic water and receive the gas comprising hydrogen from the electrolysis module. The humidifying cup is stacked above the water tank. The humidifying cup has a filter chamber and a humidifying chamber for accommodating a replenishing water. The filter is accommodated in the filter chamber to filter the gas comprising hydrogen flowing through the filter chamber. Wherein, the gas comprising hydrogen is transferred among the hydrogen water cup, the automatic diversion device, the condensation filter device, the humidifying cup and the filter by the integrated channel device. Wherein, the automatic diversion device, the condensation filter device and the humidifying cup are directly coupled to the lower cover.

Wherein, the condensation filter device comprises a condensation flow channel, and the lower cover comprises a condensation connecting channel coupled to the condensation flow channel. The humidifying cup comprises a connecting chamber coupling the water tank and the condensation connecting channel. Wherein, the humidifying chamber, the connecting chamber and the filter chamber of the humidifying cup are separated from each other.

Wherein, the hydrogen generator further comprises an electrolyte filtering module configured in the connecting chamber of the humidifying cup. The electrolyte filtering module has a continuous upward slope channel to receive and filter the gas comprising hydrogen from the water tank.

Wherein, the hydrogen generator further comprises an ozone generator. Wherein, the ozone generator is coupled to a gas flow channel which is formed by the integrated channel device, the electrolysis module, the hydrogen water cup and the automatic diversion device for the gas comprising hydrogen to flow therein. Wherein, the ozone generator is configured to generate an ozone gas into the gas flow channel to sterilize the gas flow channel when the electrolysis module stops electrolyzing.

Wherein, the integrated channel device, the electrolysis module, the hydrogen water cup and the automatic diversion device form a gas flow channel for the gas comprising hydrogen to flow therein, and the gas flow channel is configured to be connected to an ozone generator out of the hydrogen generator, so as to receive an ozone gas generated by the ozone generator from the outside to sterilize the gas flow channel when the electrolysis module stops electrolyzing.

Wherein, the hydrogen generator further comprises an ultraviolet light generator coupled to a gas flow channel formed by the integrated channel device, the electrolysis module, the hydrogen water cup and the automatic diversion device. Wherein the ultraviolet light generator is configured to generate an ultraviolet light onto the gas flow channel to sterilize the gas in the gas flow channel.

Furthermore, the present invention provides another hydrogen generator with self-sterilization function comprises an electrolysis module, a water tank, a condensation filter device, a humidifying cup and an integrated channel. The electrolysis module is configured to electrolyze an electrolytic water to generate a gas comprising hydrogen. The water tank is configured to accommodate the electrolytic water and the electrolysis module. The water tank is configured to receive the gas comprising hydrogen from the electrolysis module. The condensation filter device is stacked above the water tank and configured to receive and filter the gas comprising hydrogen. The humidifying cup is stacked above the water tank. The humidifying cup is configured to accommodate a replenishing water. The humidifying cup is configured to receive and humidify the gas comprising hydrogen from the condensation filter device. The integrated channel device is stacked above the water tank and respectively coupled to the condensation filter device and the humidifying cup. Wherein, the gas comprising hydrogen is transferred between the condensation filter device and the humidifying cup through the integrated channel device. Wherein, the pH value of the electrolytic water in the electrolysis module is in a range between 12-14 to generate a strong alkaline environment for sterilization.

Wherein, the hydrogen generator further comprises a filtering and sterilizing bottle detachably coupled to a gas output port of the hydrogen generator for filtering germs in the gas comprising hydrogen outputted from the gas output port.

Wherein, the hydrogen generator further comprises an ozone generator. Wherein, the hydrogen generator has a gas flow channel for the gas comprising hydrogen to flow therein, and the ozone generator is coupled to the gas flow channel; the ozone generator is configured to generate an ozone into the gas flow channel to sterilize the gas flow channel when the electrolysis module stops electrolyzing.

Wherein, the hydrogen generator further has a gas flow channel for the gas comprising hydrogen to flow therein, and the gas flow channel is connected to an ozone generator out of the hydrogen generator to receive an ozone gas generated by the ozone generator from the outside to sterilize the gas flow channel when the electrolysis module stops electrolyzing.

Wherein, the hydrogen generator further comprises an ultraviolet light generator coupled to a gas flow channel of the hydrogen generator. Wherein the ultraviolet light generator is configured to generate an ultraviolet light onto the gas flow channel to sterilize the gas in the gas flow channel.

Wherein, the pH value of the electrolytic water in the electrolysis module is in a range between 13-13.9.

Furthermore, the present invention provides another hydrogen generator with self-sterilization function comprising an electrolysis module and a water tank. The electrolysis module is configured to electrolyze an electrolytic water to generate a gas comprising hydrogen. The water tank is configured to accommodate the electrolytic water and coupled to the electrolysis module. The water tank is configured to provide the electrolytic water to the electrolysis module and receive the gas comprising hydrogen from the electrolysis module. Wherein, the electrolytic water in the electrolysis module comprises electrolytes with 0.5%-15% weight percentage concentration or volume percentage concentration, so as to form a strong alkaline environment with pH value in a range between 12-14 in the electrolysis module for sterilization.

Wherein, the hydrogen generator further comprises a gas flow channel for receiving the gas comprising hydrogen to flow therein. The gas flow channel comprises a gas output port to output the gas comprising hydrogen. The hydrogen generator further comprises a filtering and sterilizing bottle coupled to the gas output port for filtering germs in the gas comprising hydrogen output from the gas output port.

Wherein, the hydrogen generator further comprises a gas flow channel for receiving the gas comprising hydrogen to flow therein, and an ozone generator coupled to the gas flow channel. Wherein, the ozone generator is configured to generate an ozone gas into the gas flow channel to sterilize the gas flow channel when the electrolysis module stops electrolyzing.

Wherein, the hydrogen generator further comprises a gas flow channel for receiving the gas comprising hydrogen to flow therein. Wherein, the gas flow channel is configured to be connected to an ozone generator out of the hydrogen generator, so as to receive an ozone gas generated by the ozone generator from the outside to sterilize the gas flow channel.

Wherein, the hydrogen generator further comprises a gas flow channel for receiving the gas comprising hydrogen to flow therein, the hydrogen generator further comprising an ultraviolet light generator coupled to the gas flow channel. Wherein, the ultraviolet light generator is configured to generate an ultraviolet light onto the gas flow channel to sterilize the gas in the gas flow channel.

Wherein, the pH value of the electrolytic water in the electrolysis module is in a range between 13-13.9.

Wherein, the electrolytic water in the electrolysis module comprises electrolytes with 3% weight percentage concentration or volume percentage concentration, so as to form a strong alkaline environment with pH value in 13.88 in the electrolysis module for sterilization.

Compared with the prior art, the hydrogen generator of the present invention has the following advantages: 1. The hydrogen generator of the present invention has the automatic diversion device, which can selectively allow the gas comprising hydrogen to enter into the liquid in the hydrogen water cup according to the diversion signal. Therefore, the hydrogen generator can be adjusted to the night mode without allowing the gas comprising hydrogen to enter into the hydrogen water cup, so as to eliminate the low-frequency sound when the gas comprising hydrogen flows into the hydrogen water cup. 2. The hydrogen generator of the present invention has the pressure sensor to sense whether the flow channel for transferring the gas comprising hydrogen is smooth. When the user squeezes the pipeline, the pressure sensor will detect the difference of the gas pressure value in the gas flow channel and report to the monitoring device, and the monitoring device will control the operation of the electrolysis module to avoid danger. 3. The hydrogen generator of the present invention has the valve component to adjust the timing of the water replenishment to ensure that the gas comprising hydrogen and the replenishing water can flow smoothly in the flow channel, and to ensure that the hydrogen generator is safe during the gas production and water replenishment processes. 4. The hydrogen generator of the present invention can not only filter impurities in the gas comprising hydrogen, but also can further filter out microorganisms in the gas comprising hydrogen to ensure that the liquid comprising hydrogen and the gas comprising hydrogen are safe for the human body. 5. The hydrogen water cup of the present invention has the micro-bubble gas outlet structure, which has a micro gas outlet channel with a hollow truncated cone structure, can make the fine gas comprising hydrogen form micro-bubbles in the liquid and evenly disperse in the liquid to form liquid comprising hydrogen, so as to increase the contact area in the liquid and increase the concentration of the gas comprising hydrogen in the liquid. 6. The micro-bubble gas outlet structure of the present invention is coupled to the micro filter elements. Therefore, the gas comprising hydrogen can be filtered again before being injected into the liquid to ensure the quality. 7. The alkaline electrolysis environment of the hydrogen generator of the present invention and the ozone generators, the ultraviolet light generators, and the filter sterilization tanks can perform the hydrogen generator the self-sterilizing function, so as to maintain safety after long-term use and reduce the maintenance cost of the hydrogen generator.

### BRIEF DESCRIPTION OF THE APPENDED DRAWINGS

FIG. 1 is a functional block diagram illustrating the hydrogen generator with self-sterilization function according to an embodiment of the present invention.
FIG. 2A is an exploded diagram illustrating the hydrogen generator with self-sterilization function of FIG. 1.
FIG. 2B is a sectional diagram illustrating the electrolyte filter module of FIG. 2A.
FIG. 2C is a top view illustrating the integrated channel device of FIG. 2A.
FIG. 2D is an exploded diagram illustrating the integrated channel device of FIG. 2C.
FIG. 2E is a schematic diagram illustrating the hydrogen generator with self-sterilization function according to another embodiment of the present invention.
FIG. 3 is a top view illustrating the hydrogen generator of FIG. 2.
FIG. 4 is a schematic diagram of the flow direction of the gas comprising hydrogen in the hydrogen generator of FIG. 1.
FIG. 5A is a sectional diagram illustrating the section A-A of the hydrogen generator of FIG. 3.
FIG. 5B is a schematic diagram illustrating the filter chamber and the filter of the hydrogen generator of FIG. 5A.
FIG. 6 is a partially enlarged schematic diagram of the circled area of the hydrogen generator of FIG. 5A.
FIG. 7 is a functional block diagram illustrating the automatic diversion device of the hydrogen generator with self-sterilization function according to another one embodiment of the present invention.
FIG. 8A is a schematic diagram of the hydrogen generator of FIG. 7 under normal operating condition.
FIG. 8B is a schematic diagram of the hydrogen generator of FIG. 7 after receiving the diversion signal.
FIG. 9 is a functional block diagram illustrating the hydrogen generator according another one embodiment of the present invention.
FIG. 10 is a functional block diagram illustrating the valve component of the hydrogen generator with self-sterilization function according to another one embodiment of the present invention.
FIG. 11 is a schematic diagram of the valve component of the hydrogen generator of FIG. 10 under normal operating condition.
FIG. 12 is a schematic diagram of the appearance of the fining device of the hydrogen generator with self-sterilization function according to the embodiment of the present invention.
FIG. 13 is an exploded diagram illustrating the fining device of FIG. 12.
FIG. 14 is a functional block diagram illustrating the valve component of the hydrogen generator with self-sterilization function according to another embodiment of the present invention.
FIG. 15 is a schematic diagram of the valve component of the hydrogen generator of FIG. 14 under normal operating condition.
FIG. 16A is a sectional diagram illustrating the section B-B' of the hydrogen water cup of FIG. 2.
FIG. 16B is a sectional diagram along the section B-B' of FIG. 16A.
FIG. 17 is a partially enlarged schematic diagram of the circled area C of FIG. 16B.
FIG. 18 is an exploded diagram illustrating the gas injection component 83 of FIG. 16B.
FIG. 19 is a sectional diagram illustrating the water injection component of FIG. 18.
FIG. 20 is a schematic diagram illustrating the hydrogen generator with self-sterilization function according to another embodiment of the present invention.

### DETAILED DESCRIPTION OF THE INVENTION

For the sake of the advantages, spirits and features of the present invention can be understood more easily and clearly, the detailed descriptions and discussions will be made later by way of the embodiments and with reference of the diagrams. It is worth noting that these embodiments are merely representative embodiments of the present invention, wherein the specific methods, devices, conditions, materials and the like are not limited to the embodiments of the present invention or corresponding embodiments. Moreover, the devices in the figures are only used to express their corresponding positions and are not drawing according to their actual proportion.

In the description of the present invention, it is to be understood that the orientations or positional relationships of the terms "longitudinal, lateral, upper, lower, front, rear, left, right, top, bottom, inner, outer" and the like are based on the orientation or positional relationship shown in the drawings. It is merely for the convenience of the description of the present invention and the description of the present invention, and is not intended to indicate or imply that the device or component referred to has a specific orientation, is constructed and operated in a specific orientation, and therefore cannot be understood as limitations of the invention.

In the description of this specification, the description with reference to the terms "a specific embodiment", "another specific embodiment" or "parts of specific embodiments" etc. means that the specific feature, structure, material or feature described in conjunction with the embodiment include in at least one embodiment of the present invention. In this specification, the schematic representations of the above-mentioned terms do not necessarily refer to the same embodiment. Moreover, the described specific features, structures, materials or characteristics can be combined in any one or more embodiments in a suitable manner.

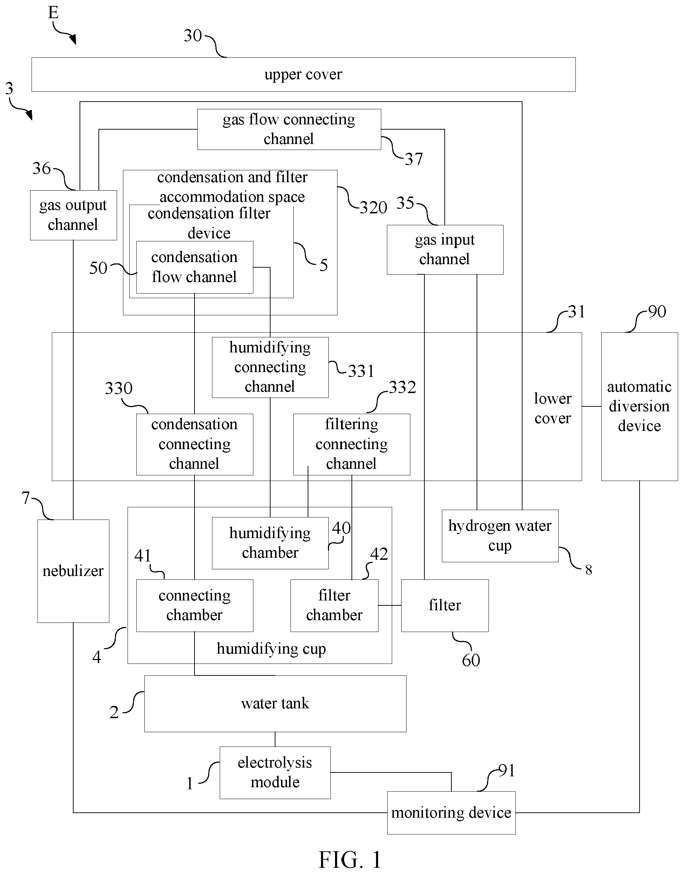

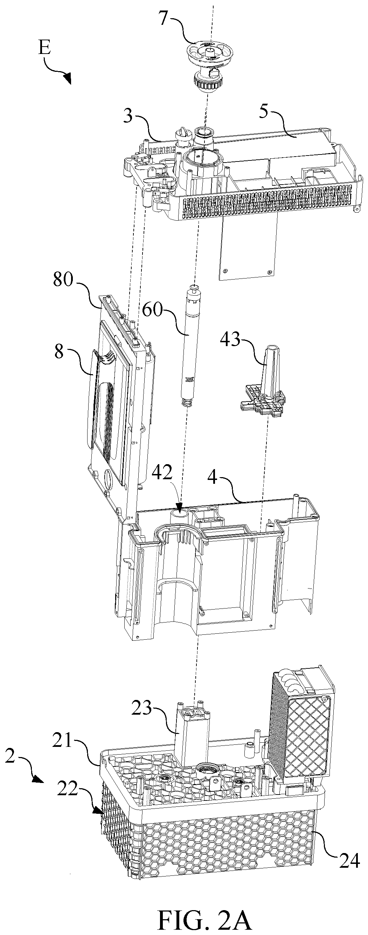

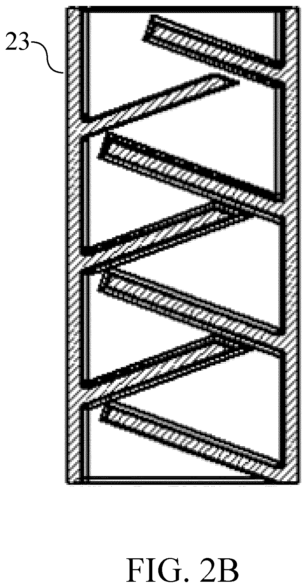

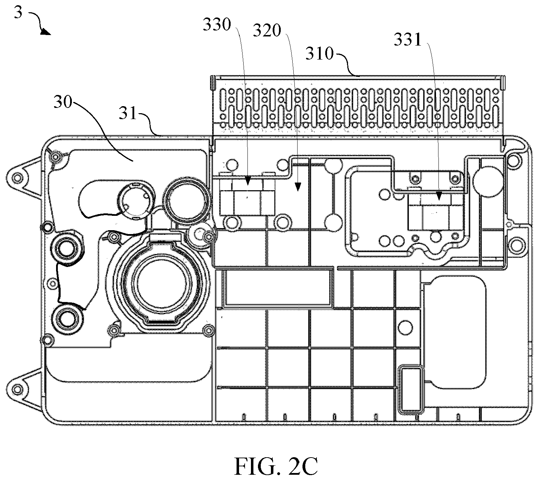

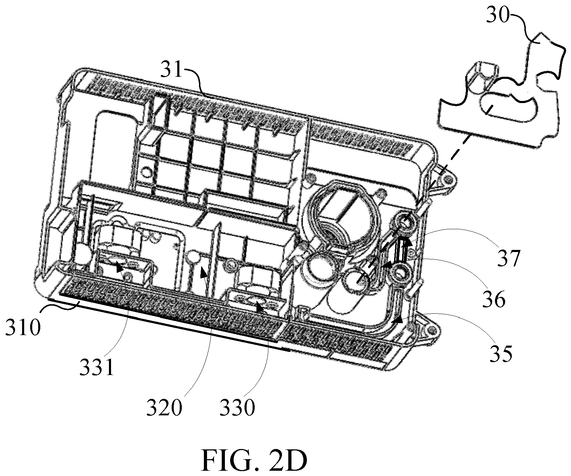

Please refer to FIG. 1, FIG. 2A to FIG. 2D. FIG. 1 is a functional block diagram illustrating the hydrogen generator E with self-sterilization function according to an embodiment of the present invention. FIG. 2A is an exploded diagram illustrating the hydrogen generator E with self-sterilization function of FIG. 1. FIG. 2B is a sectional diagram illustrating the electrolyte filter module 23 of FIG. 2A. FIG. 2C is a top view illustrating the integrated channel device 3 of FIG. 2A. FIG. 2D is an exploded diagram illustrating the integrated channel device 3 of FIG. 2C. As shown in FIG. 1 and FIG. 2A, the hydrogen generator E includes an electrolysis module 1, a water tank 2, an integrated channel device 3, a humidifying cup 4, a condensation filter device 5, a filter 6, a nebulizer?, a hydrogen water cup 8 and a frame 80 for fixing the hydrogen water cup 8. The electrolysis module 1 is arranged in the water tank 2.The water tank 2 includes a water tank body 21 and a water tank upper cover 22, and an electrolyte filter module 23 is located above the water tank upper cover 22. Wherein, as shown in FIG. 2B, the electrolyte filter module includes one or a plurality of steel wools and polyester synthetic fiber with cottons which can be placed at an angle and spaced upward one by one to form a continuous upward slope channel. The humidifying cup and the condensation filter device 5 can be stack above the water tank 2. The electrolysis module 1 is configured to electrolyze an electrolytic water to generate a gas comprising hydrogen, which contains part of hydrogen and part of oxygen (such as about 66% hydrogen and about 33% oxygen) or 100% hydrogen in other embodiments. The water tank 2 is configured to accommodate the electrolytic water and receive the gas comprising hydrogen from the electrolysis module 1 and then output the gas comprising hydrogen through the electrolyte filter module 23. The electrolyte filter module 23 has a continuous upward slope channel and includes one or a plurality of steel wools and polyester synthetic fiber with cottons, so as to filter the electrolytes in the gas comprising hydrogen and block the part components of the liquid in the gas comprising hydrogen by the electrolyte filter module23. As shown in FIG. 2C and FIG. 2D, the integrated channel device 3 includes a upper cover 30 and a lower cover 31, and the gas input channel 35, the gas output channel 36 and the gas flow connecting channel 37 are arranged between the upper cover 30 and the lower cover 31. The lower cover 31 is an integrally formed structure, which includes one-piece injection molding or using welding to integrate different components into one body to form a one-piece molded structure. In practical application, as shown in FIG. 1, FIG.2A to FIG. 2D, the integrated channel device 3 is vertically stacked above the humidifying cup 4, and the humidifying cup 4 is vertically stacked above the water tank 2.

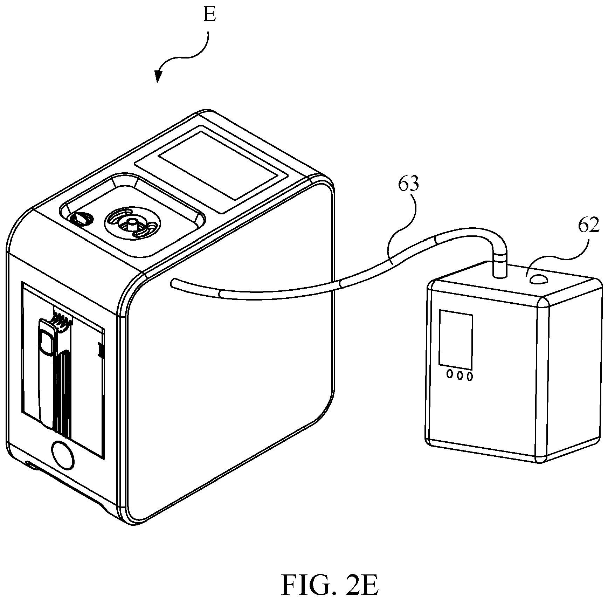

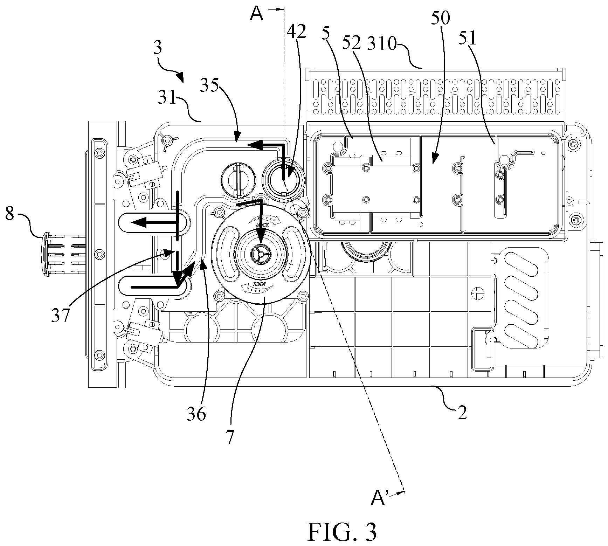

Please refer to FIG. 3 together. FIG. 3 is a top view illustrating the hydrogen generator E of FIG. 2. Wherein, the lower cover 31 has a condensation and filter accommodation space 320 for accommodating the condensation filter device 5. The humidifying cup 4 is stacked between the integrated channel device 3 and the water tank 2, and embedded the lower cover 31. The condensation filter device 5 is configured to condense and filter the gas comprising hydrogen. The condensation filter device 5 can have a condensation flow channel 50. In practical application, the condensation filter device 5 can be embedded into the integrated channel device 3 and can be designed with design for easy replacement. The filter 60 is configured to filter the gas comprising hydrogen. The nebulizer 7 is embedded the lower cover 31 and coupled to the gas output channel 36 to receive the gas comprising hydrogen. The nebulizer 7 generates an atomized gas to be mixed with the gas comprising hydrogen to form a healthy gas. The hydrogen water cup 8 is configured to accommodate drinking water, and injected the gas comprising hydrogen into the drinking water to generate water comprising hydrogen. In practical application, the hydrogen water cup 8 can be embedded into the frame 80 and then coupled (or directly connected) to the integrated channel device 3. When the hydrogen water cup 8 is separated from the frame 80 and is not coupled to the integrated channel device 3, the electrolysis module 1 will stop operating. Wherein, the gas input channel 35 and the gas output channel 36 can be selectively coupled to the hydrogen water cup 8, and the gas flow connecting channel 37 can be selectively coupled to the gas input channel 35 and the gas output channel 36.

In this way, the gas comprising hydrogen can be transferred between the humidifying cup 4, the condensation filter device 5, the filter 60, the nebulizer 7 and the hydrogen water cup 8 by the integrated channel device 3. In one embodiment, the humidifying cup 4, the condensation filter device 5 and the nebulizer 7 can be directly coupled to the lower cover 31. Furthermore, the hydrogen water cup 8 can also be directly coupled to the lower cover 31.

In one embodiment, the electrolysis module 1 can be arranged in the water tank 2 and can receive the electrolytic water from the water tank 2 to generate the gas comprising hydrogen. When the electrolysis module 1 electrolyzes the electrolytic water, the electrolysis module 1 directly generates the gas comprising hydrogen in the water tank 2. In practical application, the outer surface of the water tank 2 can be a honeycomb structure 24 to increase the rigidity of the water tank 2 to avoid deforming by stretching the water tank 2 which is caused by the gas comprising hydrogen. In addition, the gas comprising hydrogen can easily move toward the connecting chamber 41 due to the rigidity of the honeycomb structure 24 of the water tank 2, so as to avoid the gas comprising hydrogen stagnating in the water tank 2 to burst the water tank 2.

The humidifying cup 4 includes a humidifying chamber 40, a connecting chamber 41, and a filter chamber 42. The humidifying chamber 40 is configured to accommodate a replenishing water to humidify the gas comprising hydrogen. The connecting chamber 41 is configured to connect to the water tank 2 and the integrated channel device 3 for the gas comprising hydrogen entering into the condensation filter device 5. In this embodiment, the electrolyte filter module 23 can be arranged in the connecting chamber 41, and the gas comprising hydrogen can be filtered once by the electrolyte filter module 23 before entering into the condensation flow channel 50. The filter chamber 42 is configured to accommodate the filter 60. The filter 60 filters the gas comprising hydrogen while flowing through the filter chamber 42. Wherein, the humidifying chamber 40, the connecting chamber 41, and the filter chamber 42 are separated from each other. In addition, the lower cover 31 of the integrated channel device 3 further includes a condensation connecting channel 330, a humidifying connecting channel 331 and a filtering connecting channel 332. The condensation connecting channel 330 is configured to connect the water tank 2 and the condensation filter device 5 through the connecting chamber 41. The humidifying connecting channel 331 is configured to connect the condensation flow channel 50 and the humidifying chamber 40. The filtering connecting channel 332 is configured to connect the humidifying chamber 40 and the filter chamber 42. The filter chamber 42 couples with the gas input channel 35 to output the gas comprising hydrogen after filtering.

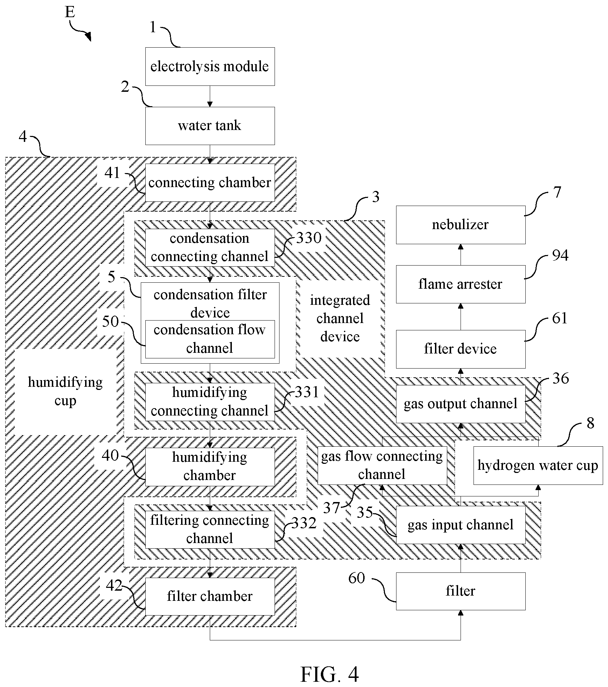

In detail, the integrated channel device 3 stacks and embeds other components, so as to provide a gas path in the hydrogen generator E of the present invention in FIG. 1 for the gas comprising hydrogen flowing therein. In order to explain more clearly the flow direction of the gas comprising hydrogen, please refer to FIG. 4. FIG. 4 is a schematic diagram of the flow direction of the gas comprising hydrogen in the hydrogen generator E of FIG. 1. As shown in FIG. 4, the electrolysis module 1 electrolyzes the electrolytic water to generate a gas comprising hydrogen. Since the electrolysis module 1 can be arranged in the water tank 2, the gas comprising hydrogen will be output in the water tank 2 from the electrolysis module 1. Next, the gas comprising hydrogen sequentially flows through the connecting chamber 41 of the humidifying cup 4, the condensation connecting channel 330 of the integrated channel device 3, the condensation flow channel 50 of the condensation filter device 5, the humidifying connecting channel 331 of the integrated channel device 3, the humidifying chamber 40 of the humidifying cup 4, the filtering connecting channel 332 of the integrated channel device 3, the filter chamber 42 of the humidifying cup 4, the filter 60, the gas input channel 35 of the integrated channel device 3, the gas output channel 36, the flame arrester 94, and the nebulizer 7. Wherein, the gas comprising hydrogen between the gas input channel 35 and the gas output channel 36 can selectively flow through the hydrogen water cup 8 or the gas flow connecting channel 37 of the integrated channel device 3. However, it should be understood that the above-mentioned flow direction of the gas comprising hydrogen is one of the embodiment of the hydrogen generator E of the present invention. Person having ordinary skill in the art can adjust the order of each component according to design or requirement, but is not limited thereto.

In the above-mentioned embodiment, the pH value of the electrolytic water in the electrolysis module 1 and the water tank 2 is in a range between 12-14 to generate a strong alkaline environment. The strong alkaline environment in the electrolytic water can sterilize and disinfect the water tank 2 and the electrolysis module 1 at the same time, so as to protect the gas comprising hydrogen from infecting by germs. On the other hand, the internal temperature of the electrolysis module 1 is in a range between 50°C to 80°C while the electrolysis module 1 performs electrolysis. However, the internal temperature of the electrolysis module 1 is too high to perform electrolysis well. Therefore, in practice, the internal temperature of the electrolysis module 1 can maintain at about 60°C during electrolysis.

The gas comprising hydrogen, which is generated by the electrolysis module 1, enters into the water tank 2, and then flows through the condensation filter device 5 and the integrated channel device 3. Since the gas comprising hydrogen carries the alkaline moisture from the electrolytic water, the flow path, which the gas comprising hydrogen flows through can be sterilized and disinfected at the same time. Not only the electrolysis module 1 and the water tank 2 of the hydrogen generator E provide the functions with self-disinfection and self-sterilization, but also the flow path which the gas comprising hydrogen flows through provides the same effect. Therefore, the hydrogen generator E of the present invention can protect the gas comprising hydrogen from infecting by germs, so as to provide users more safely to inhale the health caring gas or the gas comprising hydrogen.

In addition to the self-sterilization function made by the above-mentioned with alkaline environment and electrolysis temperature, in another embodiment, the hydrogen generator E can further include a filter device 61, to filter microorganisms in the gas comprising hydrogen or kill bacteria in the gas comprising hydrogen. The components in the filter device 61 can include at least one of activated carbon, nanosilver sputtering, polyethylene terephthalate (PET), and polypropylene (PP) fiber cloth. The antibacterial types can include Staphylococcus aureus, Escherichia coli, Pseudomonas aeruginosa and Methicillin-resistant Staphylococcus aureus. It should be understood that person having ordinary skill in the art can add multiple filter device 61 and adjust the positions of the filter device 61 as needed, but is not limited thereto. The filter 61 can be seen as a disposable replacement component and can be placed in front of the flame arrester 94 (as shown in FIG. 4), or can be placed in the nebulizer 7 or at the outlet of the nebulizer 7.

The above-mentioned filter can be a device with any type, and can be arranged at different positions on the hydrogen generator E or outside of the hydrogen generator E. In one embodiment, the hydrogen generator E can include an ozone generator. The ozone generator couples to a gas flow channel which is formed between the integrated channel device, the electrolysis module, the hydrogen water cup and the automatic diversion device for the gas comprising hydrogen to flow therein. When the electrolysis module 1 stops electrolyzing, the ozone generator generates an ozone gas into the gas flow channel to sterilize the gas flow channel. In this way, when the electrolysis module 1 starts operating again, the gas flow channel has been cleaned and is in a sterile or germ-free state. At this time, the gas comprising hydrogen generated by the electrolysis module 1 will not be infected by germs in the gas flow channel. Furthermore, the ozone generator can also be coupled in the liquid flow channel to provide the ozone gas to sterilize.

In the previous embodiment, the ozone generator is arranged in inside the hydrogen generator E. In practice, the ozone generator can also be arranged in outside the hydrogen generator E. Please refer to FIG. 2E. FIG. 2E is a schematic diagram illustrating the hydrogen generator E with self-sterilization function according to another embodiment of the present invention. As shown in FIG. 2E, the hydrogen generator E can further include a case for accommodating the units aforementioned. The ozone generator 62 can be coupled to a gas flow channel, which is formed between the integrated channel device, the electrolysis module, the hydrogen water cup and the automatic diversion device for the gas comprising hydrogen to flow therein, through the conveyor pipe 63 or coupled to the liquid flow channel. Therefore, when the electrolysis module stops electrolyzing, the ozone generator 62 outside the hydrogen generator E can generate ozone gas. Next, the gas flow channel or the liquid flow channel of the hydrogen generator E can be sterilized and disinfected by the ozone gas while the ozone gas flowing through the conveyor pipe 63.

In addition to the ozone generator, the hydrogen generator of the present invention can also include different device with sterilization or disinfection function. In another embodiment, the hydrogen generator E further includes an ultraviolet light couples to the gas flow channel between the integrated channel device, the electrolysis module, the hydrogen water cup and the automatic diversion device. Moreover, the ultraviolet light can couples to any gas flow channel or any liquid flow channel in the hydrogen generator E. The ultraviolet light can generate an ultraviolet light onto the gas flow channel or liquid flow channel when the electrolysis module 1 is electrolyzing or stops electrolyzing. The ultraviolet light sterilizes and disinfects with its wavelength band. Therefore, when the electrolysis module 1 is electrolyzing or stops electrolyzing, the ultraviolet light provides the ultraviolet light onto the gas flow channel or liquid flow channel to sterilize and disinfect the gas comprising hydrogen or water or the entire gas flow channel or the entire liquid flow channel and each module in the hydrogen generator E.

In all above-mentioned embodiments provide sterilization and disinfection functions in the hydrogen generator E, but in practice, the health caring gas or the gas comprising hydrogen after outputting can also be sterilized and disinfected to provide more complete protection. In another embodiment, the hydrogen generator E can further include a filtering and sterilizing bottle detachably coupled to the gas output port of the hydrogen generator E for filtering germs in the gas comprising hydrogen outputted from the gas output port. In detail, in some of the above-mentioned embodiments, the hydrogen generator E has the nebulizer 7. After the gas comprising hydrogen enters into the nebulizer 7, the gas comprising hydrogen will be mixed with an atomized gas generated by the nebulizer 7 to form a healthy gas and then output from the outside of hydrogen generator E. At the same time, the filtering and sterilizing bottle sterilizes and disinfects the healthy gas in the gas output port of the hydrogen generator E. In other embodiments, the hydrogen generator E does not include the nebulizer. At this same time, the hydrogen generator E will directly output the gas comprising hydrogen, and the gas comprising hydrogen will be sterilized and disinfected in the gas output port of the hydrogen generator E by the filtering and sterilizing bottle.

In summary, the strong alkaline environment and the operating temperature in the electrolysis module and the water tank of the hydrogen generator E with self-sterilization function of the present invention that is not conducive to the survival of germs or bacteria. Therefore, the gas comprising hydrogen generated by the hydrogen generator E of the present invention is not infected by germs. In addition, the gas comprising hydrogen carries the alkaline moisture from the electrolysis module and the water tank to sterilize and disinfect in the gas flow channel while the gas comprising hydrogen flowing through the gas flow channel. In this way, the interior of the hydrogen generator E is sterile or has few bacteria. On the other hand, different devices such as ozone generators, ultraviolet light generators, and filtering and sterilizing bottles can also be arranged inside or outside of the hydrogen generator E to further sterilize and disinfect the gas comprising hydrogen, the health caring gas, the gas in the hydrogen generator E, the gas flow channel, the liquid flow channel, and various modules. Through the above technical means, the hydrogen generator of the present invention continuously performs self-disinfection and self-sterilization without being disassembled. Therefore, the hydrogen generator of the present invention not only provide users with germ-free, the pure gas comprising hydrogen or the health caring gas, but also significantly reduce the maintenance cost of the hydrogen generator.

The flame arrester 94 can include at least one of a metal mesh filter element and a corrugated filter element in one embodiment. The metal mesh filter element can be stainless steel or copper mesh with a diameter of 0.23-0.315mm and composed with multiple layers. The corrugated filter element can be stainless steel, copper-nickel alloy, aluminum, and aluminum alloy to prevent the violent flame of deflagration and withstand the corresponding thermal and mechanical effects. The flame arrestor 94 is configured to block off the fire from flowing to the flame arrestor 94, thereby isolating the two spaces to avoid the fire spreading from one side of the flame arrester to the other side, which can cause the fire to spread through the gas flow path and make an explosion. In this embodiment, the flame arrestor 94 is arranged between the nebulizer 7 and the gas output channel 36. In addition to using the flame arrestor 94 to prevent the spread of fire, the hydrogen generator E of the present invention can also use the replenishing water in the humidifying chamber 40 and the electrolytic water in the water tank 2 to achieve arresting fire with multi-zone. In detail, the hydrogen generator E can be divided into three zones, such as from the water tank 2 to the humidifying chamber 40, the humidifying chamber 40 to the flame arrestor 94, and the flame arrestor 94 to the nebulizer 7 (even extending to the user) by the replenishing water and the electrolytic water. When the fire enters the inside of the hydrogen generator E from the nebulizer 7, the fire will be blocked by the flame arrestor 94. When the fire occurs from the gas flow channel between the humidifying chamber 40 and the flame arrestor 94, the fire will be blocked by the replenishing water of the humidifying chamber 40 and the flame arrestor 94. When the fire occurs from the electrolysis module 1, the fire will be blocked by the electrolytic water of the water tank 2. In addition to achieving arresting fire with multi-zone, the hydrogen generator E of the present invention can also achieve arresting fire with multi-stage. For example, when the fire enters the inside of the hydrogen generator E from the nebulizer 7 and the flame arrestor 94 cannot block the fire, the replenishing water in the humidifying chamber 40 can be the second stage for arresting the fire. In this way, the safety of the hydrogen generator E can be fully improved while the hydrogen generator E is operating. It should be noted that person having ordinary skill in the art can add flame arresters 94 and adjust the positions of the flame arresters 94 according to the design or requirement to achieve arresting fire with more multi-zones and more multi-stages, and are not limited thereto.

Please refer to FIG. 3. As shown in FIG. 3, in order to clearly show the inside of the condensation filter device 5, the gas input channel 35, the gas output channel 36 and the gas flow connecting channel 37, the cover of the condensation filter device 5 and the upper cover 30 of the integrated channel device 3 are hidden. In FIG. 3, the relative positions of the filter chamber 42, the gas input channel 35, the gas flow connecting channel 37, the hydrogen water cup 8, the gas output channel 36 and the nebulizer 7 can be clearly seen. Wherein, the flow direction of the gas comprising hydrogen is marked with solid arrows and dotted arrows. Under normal state, the gas comprising hydrogen sequentially flows through the filter chamber 42, the gas input channel 35, the hydrogen water cup 8, the gas output channel 36 and then reaches the nebulizer 7. In the state after the diversion signal is generated, the gas comprising hydrogen sequentially flows from the gas input channel 35 through the gas flow connecting channel 37 into the gas output channel 36 along the direction of the dotted arrow.

The condensation flow channel 50 of the condensation filter device 5 is formed by a plurality of separators 51, and the condensation flow channel 50 can accommodate a filter cotton 52. The filter cotton 52 can be at least one steel wool and polyester synthetic fiber cotton. The filter cotton 52 is configured to filter the substance in the gas comprising hydrogen, such as electrolyte or alkali mist. A heat sink (not shown in FIG. 3) can be arranged on the filter cotton 52. When the filter cotton 52 is closely attached to the heat sink, the filter cotton 52 can transfer the heat energy from the gas comprising hydrogen to enhance the condensation effect. In practical application, the filter cotton 52 can be an integrally formed structure, and the filter cotton 52 has holes corresponding to the position of the separators 51. When the filter cotton 52 is embedded into the condensation flow channel 50, the filter cotton 52 can be directly coupled to the separators 51 to improve the tightness between the condensation flow channel 50 and the filter cotton 52. In this way, the gas comprising hydrogen can be ensured to be filtered and condensed in the condensation channel 50. The filter cotton 52 can also be a separate structure containing multiple pieces of fiber cotton, or can also contain a combination of one or several pieces of steel wire and one or several pieces of fiber cotton.

The lower cover 31 of the integrated channel device 3 can have a movable flip-up structure 310 to form one side surrounding the condensation and filter accommodation space 320. The condensation filter device 5 can be placed in the condensation and filter accommodation space 320 through the movable flip-up structure 310, so that the condensation filter device 5 can selectively fit into the lower cover 31. Therefore, the hydrogen generator E can be opened and closed through the movable flip-up structure 310 and users can easily replace the condensation filter device 5 in the condensation and filter accommodation space 320.

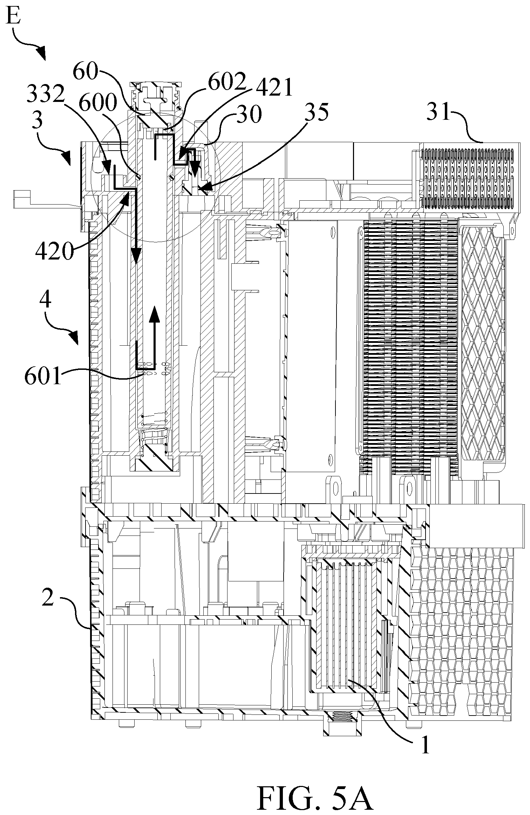

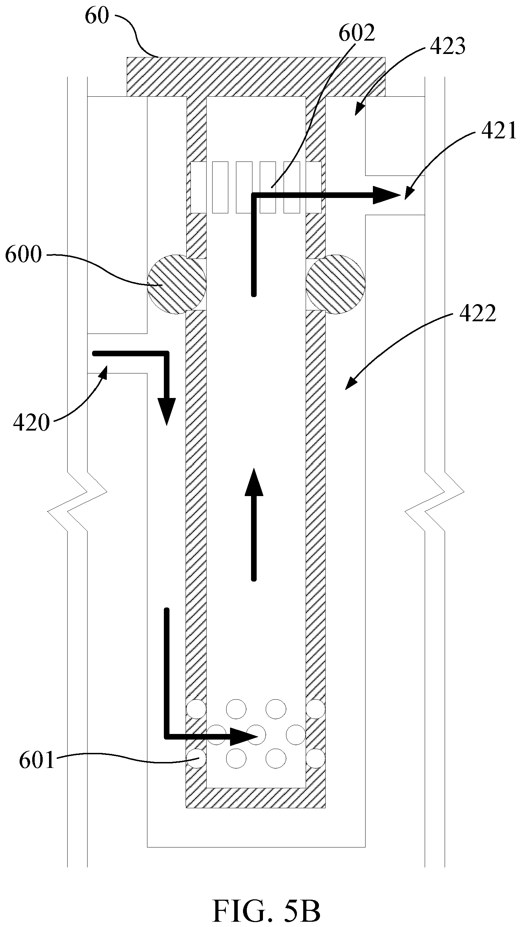

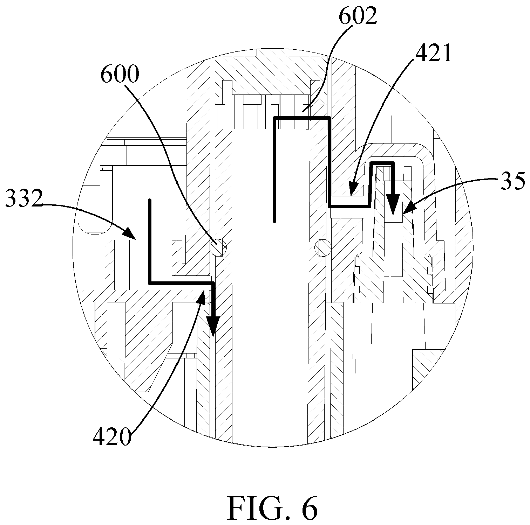

In order to clearly understand the relative position and structure between the filter 60 and the filter chamber 42, please refer to FIG. 5A to FIG. 6. FIG. 5A is a sectional diagram illustrating the section A-A of the hydrogen generator E of FIG. 3. FIG. 5B is a schematic diagram illustrating the filter chamber 42 and the filter 60 of the hydrogen generator E of FIG. 5A. FIG. 6 is a partially enlarged schematic diagram of the circled area of the hydrogen generator E of FIG. 5A. As shown in FIG. 5A to FIG. 6, the filter chamber 42 has a filter chamber inlet 420 and a filter chamber outlet 421. The filter chamber inlet 420 is coupled to the filtering connecting channel 332. The filter chamber outlet 421 is coupled to the gas input channel 35. The filter 60 includes a gas blocking ring 600, and has a plurality of filter inlets 601 and a plurality of filter outlets 602. The gas blocking ring 600 is located around the outside of the filter 60 and is configured to separate the filter chamber 42 into an unfiltered space 422 and a filtered space 423. The unfiltered space 422 is coupled to the filter chamber inlet 420 and the filter inlets 601. The filtered space 423 is coupled to the filter outlets 602 and the filter chamber outlet 421. When the gas comprising hydrogen enters into the filter chamber 42 through the filtering connecting channel 332, the gas comprising hydrogen will sequentially flow through the filter chamber inlet 420, the unfiltered space 422, the filter inlets 601 into the filter 60, and then will flow into the gas input channel 35 through the filter outlets 602, the filtered space 423 and the filter chamber outlet 421. In this way, it is unnecessary to add any pipe from outside due to structural design between the filter 60 and the filter chamber 42 can be seen as a flow channel, so as to achieve efficient space utilization and improve the sealing of the flow channel.

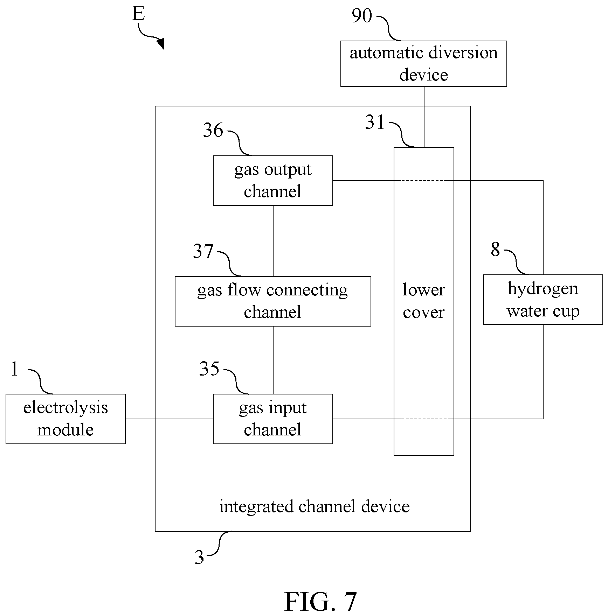

Please refer to FIG. 7. FIG. 7 is a functional block diagram illustrating the automatic diversion device 90 of the hydrogen generator E with self-sterilization function according to another one embodiment of the present invention. As shown in FIG. 7, the hydrogen generator E with self-sterilization function of the present invention further includes an automatic diversion device 90. The automatic diversion device 90 is configured to selectively connect with the gas input channel 35, the hydrogen water cup 8 and the gas output channel 36, or connect with the gas input channel 35, the gas flow connecting channel 37 and the gas output channel 36 according to the diversion signal. In practical application, the automatic diversion device 90 can be actuated by a solenoid valve.

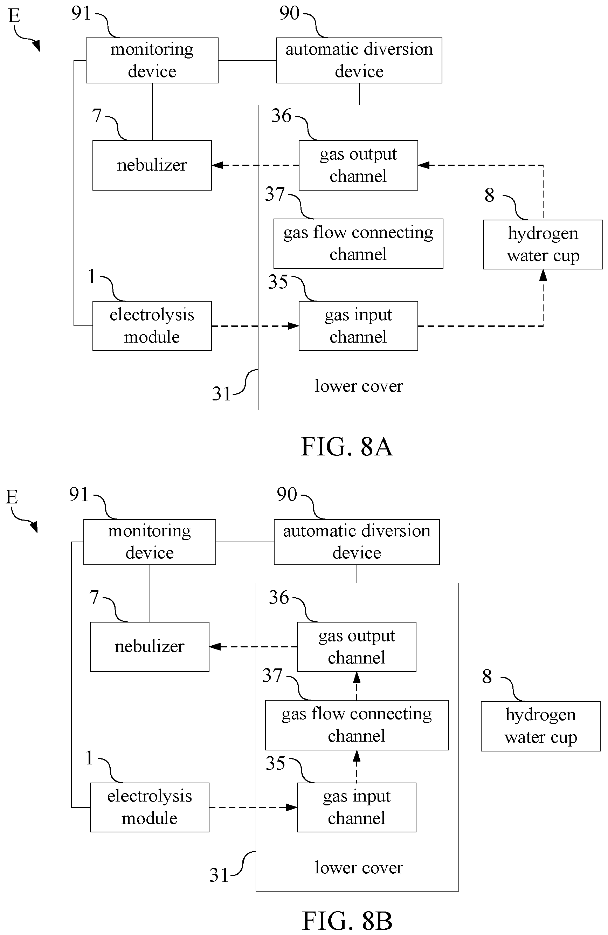

Please refer to FIG. 8A and FIG. 8B. FIG. 8A is a schematic diagram of the hydrogen generator E of FIG. 7 under normal operating condition. FIG. 8B is a schematic diagram of the hydrogen generator E of FIG. 7 after receiving the diversion signal. As shown in FIG. 7, FIG. 8A and FIG. 8B, the gas input channel 35, the gas flow connecting channel 37 and the gas output channel 36 are located in the lower cover 31. The automatic diversion device 90 is coupled to the lower cover 31, and switches different flow channel according to the diversion signal to adjust the flow direction of the gas comprising hydrogen. As shown in FIG. 8A, under normal operating condition, the monitoring device 91 can control the automatic diversion device 90 to connect with the hydrogen water cup 8 to the gas input channel 35 and the gas output channel 36, that is, the gas input channel 35 is connected to the gas output channel 36 through the hydrogen water cup 8. Therefore, the gas comprising hydrogen generated from the hydrogen generator E can flow through the gas input channel 35, the hydrogen water cup 8 and the gas output channel 36 and finally flow into the nebulizer 7 (as shown by the dotted arrow shown in FIG. 8A). When the gas comprising hydrogen flows through the hydrogen water cup 8, some of the gas comprising hydrogen will be injected into the drinking water in the hydrogen water cup 8 to form the water comprising hydrogen. Next, the gas comprising hydrogen which is not mixed with the drinking water will be output from the hydrogen water cup 8 and flow to the nebulizer 7 through the gas output channel 36. As shown in FIG. 8B, under another operating condition, the automatic diversion device 90 can connect the gas input channel 35, the gas flow connecting channel 37 and the gas output channel 36 according to the diversion signal by the monitoring device 91, and isolate the hydrogen water cup 8 from the gas input channel 35 and the gas output channel 36. At this time, the gas input channel 35 connects the gas flow connecting channel 37 and the gas output channel 36. Therefore, the gas comprising hydrogen generated from the hydrogen generator E can flow through the gas input channel 35, the gas flow connecting channel 37 and the gas output channel 36 and finally flow into the nebulizer 7 (as shown by the dotted arrow shown in FIG. 8B). In one embodiment, the gas comprising hydrogen can be transferred between the humidifying cup 4, the condensation filter device 5, the filter 6, the nebulizer 7, the hydrogen water cup 8 and the automatic diversion device 90 by the integrated channel device 3. Wherein, the humidifying cup 4, the condensation filter device 5, the nebulizer 7 and the automatic diversion device 90 can directly couples to the lower cover 31.

In practical application, both of the hydrogen water cup 8 and the nebulizer 7 generate low-frequency sound when the gas comprising hydrogen is injected into the drinking water in the hydrogen water cup 8 or when the nebulizer 7 is operating to atomize with vibration. Low-frequency sound may not be noticed in daily life, but may affect the quality when users are sleeping in the night. Therefore, the monitoring device 91 of the hydrogen generator E of the present invention couples the automatic diversion device 90 to selectively generate the diversion signal to control the automatic diversion device 90. For example, user can adjust the hydrogen generator E to the night mode in the night. At this time, the monitoring device 91 sends a diversion signal to control the automatic diversion device 90, and then the automatic diversion device 90 will control the gas flow connecting channel 37 to connect with the gas input channel 35 and the gas output channel 36, so as to make the gas comprising hydrogen do not flow into the hydrogen water cup 8. Furthermore, the monitoring device 91 can also turn the nebulizer 7 off to stop generating the atomized gas under the night mode, so as to avoid low-frequency sound generated by the nebulizer 7. In another one embodiment, after canceling the night mode, the monitoring device 91 will control the automatic diversion device 90 to let the gas comprising hydrogen flow into the hydrogen water cup 8 and control the nebulizer 7 to turn on and generate the atomized gas.

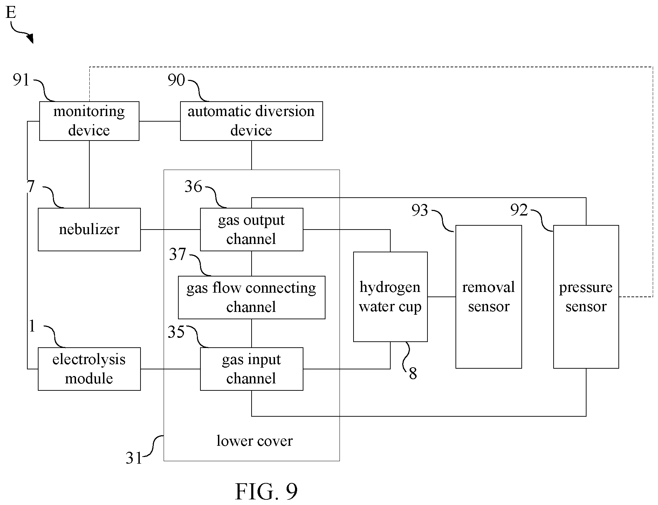

Sometimes the breathing tube for the user inhaling of the hydrogen generator E may be squeezed by the user when they change their posture. At this time, since the breathing tube is squeezed, the gas comprising hydrogen cannot be output normally in the breathing tube which will lead the air pressure in the hydrogen generator E to rise a lot and then the hydrogen generator E will be exploded or damaged. Therefore, the hydrogen generator E of the present invention further includes a pressure sensor 92 to solve this problem. Please refer to FIG. 9. FIG. 9 is a functional block diagram illustrating the hydrogen generator according another one embodiment of the present invention. As shown in FIG. 9, the pressure sensor 92 is coupled to at least one of the gas input channel 35 and the gas output channel 36. The pressure sensor 92 is configured to sense each gas pressure value of the gas input channel 35, the gas output channel 36, and generate a pressure sensing signal according to each gas pressure value. The monitoring device 91 is coupled to the pressure sensor 92 and controls the operation of the electrolysis module 1 according to the pressure sensing signal. In practical application, when the user squeezes the breathing tube, the gas comprising hydrogen in the gas channel of the breathing tube will not be able to flow out from the hydrogen generator E and leads the gas pressure to rise a lot in at least one of the gas input channel 35 and the gas output channel 36. At this time, the pressure sensor 92 senses the gas pressure value, which is detected from the gas input channel 35 or the gas output channel 36, is increasing, the pressure sensor 92 will generate the pressure sensing signal and send to the monitoring device 91 to control the electrolysis module 1. Next, the monitoring device 91 controls the electrolysis module 1 to stop generating the gas comprising hydrogen, so as to prevent the hydrogen generator E from being exploded or be damaged or prevent the components in the hydrogen generator E from being stretched by accumulating too much the gas comprising hydrogen. The components in the hydrogen generator E which have been stretched by the gas comprising hydrogen will also cause gas leakage problems when the hydrogen generator E is operating in the future.

In addition, the pressure sensor 92 not only can detect the difference of the gas pressure value caused by squeezed by the user, the pressure sensor 92 also can detect whether the gas channel in the hydrogen generator E is unblocked. Since the flame arrester 94, the filter cotton 52, the filter 60 will gradually be clogged after long-term use, the gas pressure value of the gas channel will gradually increase. Therefore, the hydrogen generator E can detect the components by the pressure sensor 92 and remind the user to replace the components.

In one embodiment, the pressure sensor 92 can also send out the pressure sensing signal with the gas pressure value at regular time intervals, and the monitoring device 91 monitors the difference of the pressure sensing signal. If the gas pressure value is larger than the upper threshold, or less than the lower threshold, or the slope of the difference of the gas pressure value is too large. At this time, the pressure sensor 92 will determine the pressure is abnormal, and the monitoring device 91 will stop the electrolysis module 1 generating the gas comprising hydrogen or will control the electrolysis module 1 to enhance production of the gas comprising hydrogen. If the user changes his posture, and the breathing tube is not be squeezed. At this time, the pressure sensor 92 will detect that the pressure value is between the upper threshold and lower threshold, or the slope of the difference of the gas pressure value is became smooth, and the monitoring device 91 will control the electrolysis module 1 to restart to generate the gas comprising hydrogen. In another one present embodiment, the hydrogen generator E further includes a pressure relief device. The monitoring device 91 can turn the electrolysis module 1 off and turn the pressure relief device on at the same time to release the pressure in the gas flow channel, thereby avoiding danger or damage the hydrogen generator.

When the hydrogen water cup 8 is detached from the lower cover 31, the gas comprising hydrogen will probably flow out from the junction between the hydrogen water cup 8 and the lower cover 31 and then interrupt the connection between the gas input channel 35, the hydrogen water cup 8 and the gas output channel 36. To solve this problem, the hydrogen generator E of the present invention further includes a removal sensor 93 coupled to the hydrogen water cup 8. Therefore, when the hydrogen water cup 8 is detached from the lower cover 31, the removal sensor 93 will generate a second diversion signal to make the gas comprising hydrogen change to flow into the gas output channel 36 through the gas flow connecting channel 37, thereby solving the problem of the gas flow channel being interrupted.

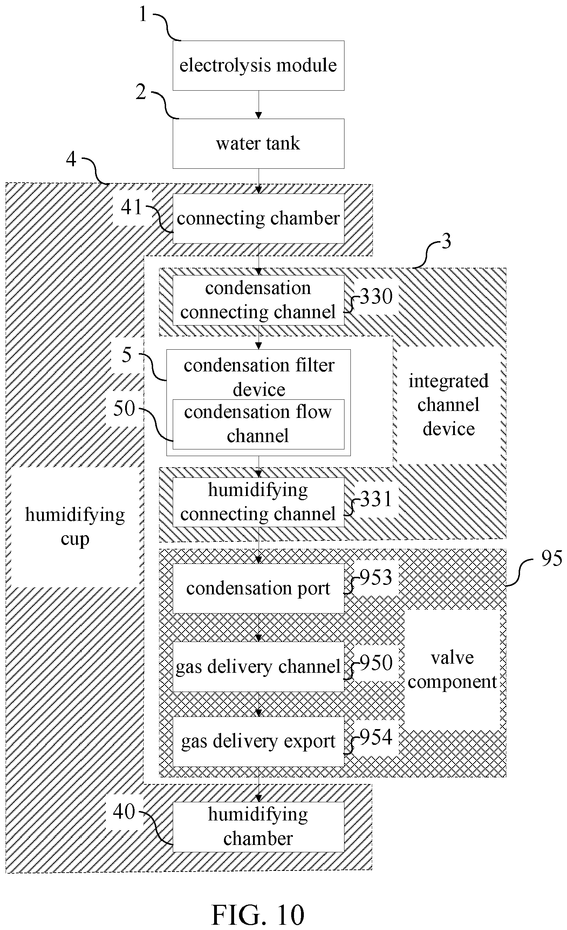

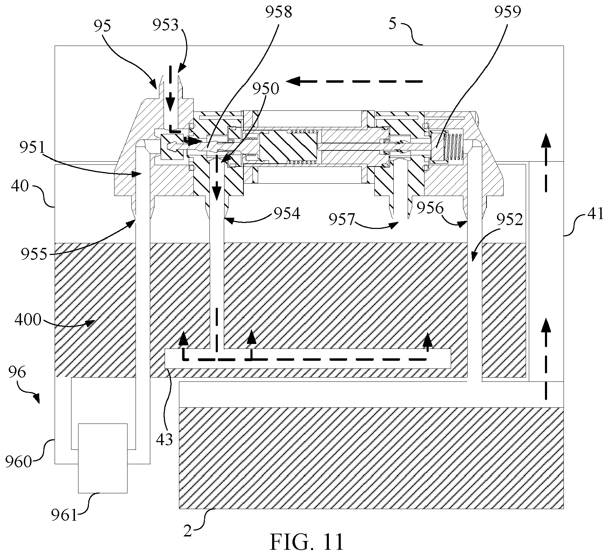

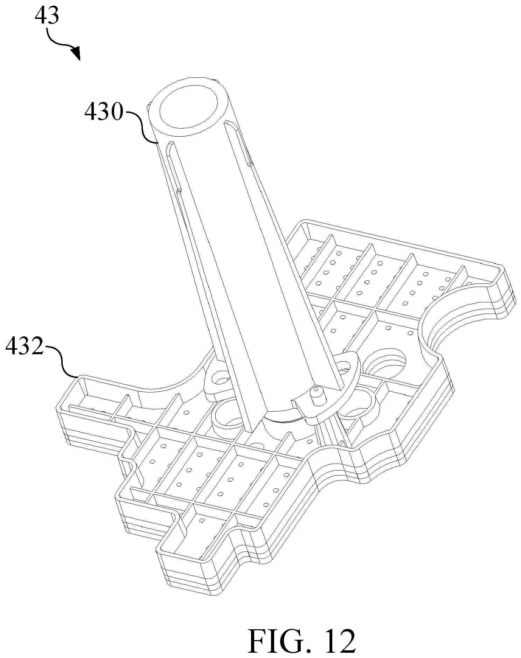

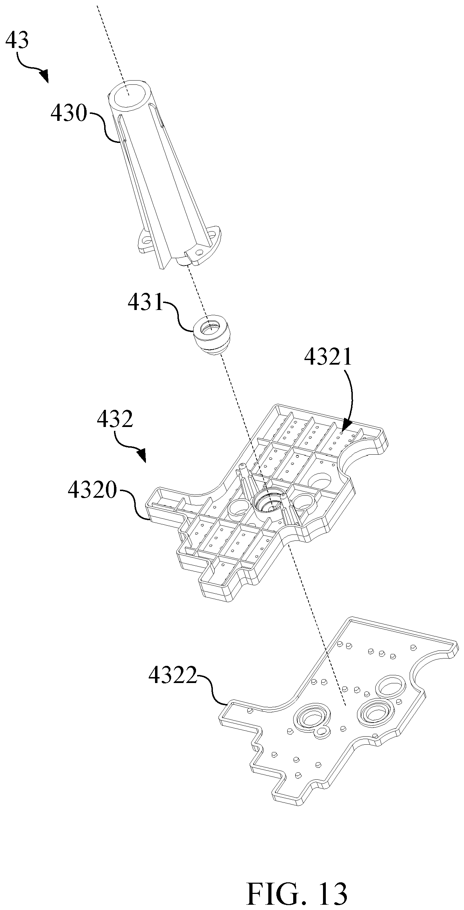

In order to extend the operating time of the hydrogen generator E to generate more gas comprising hydrogen, the hydrogen generator E of the present invention further includes a valve component 95 and a water guiding component 96. When the electrolytic water is insufficient, the replenishing water can be replenished from the humidifying chamber 40. When the replenishing water is insufficient, the user replenishes the replenishing water into the humidifying chamber 40. In order to clearly describe the valve component 95 and the water guiding component 96, it will be described into two paths, such as the gas flow path for transferring the gas comprising hydrogen and the liquid flow path for supplementing the electrolytic water. Please refer to FIG. 10 to FIG. 13. FIG. 10 is a functional block diagram illustrating the valve component 95 of the hydrogen generator E with self-sterilization function according to another one embodiment of the present invention. FIG. 11 is a schematic diagram of the valve component 95 of the hydrogen generator E of FIG. 10 under normal operating condition. FIG. 12 is a schematic diagram of the appearance of the fining device 43 of the hydrogen generator E with self-sterilization function according to the embodiment of the present invention. FIG. 13 is an exploded diagram illustrating the fining device 43 of FIG. 12. As shown in FIG. 10 to FIG. 11, the gas delivery channel 950 of the valve component 95 can be coupled to the condensation flow channel 50 of the condensation filter device 5 and the humidifying chamber 40 of the humidifying cup 4. Furthermore, the valve component 95 has a condensation port 953 and a gas delivery export 954 coupled to the gas delivery channel 950. The condensation port 953 is at least coupled to the condensation filter device 5, and the gas delivery export 954 is at least coupled to the humidifying cup 4. Wherein, the gas comprising hydrogen flows through the water tank 2, the connecting chamber 41 and the condensation filter device 5, and then flows through the condensation port 953. Next, the gas comprising hydrogen will be output into the humidifying chamber 40 of the humidifying cup 4 through the gas delivery export 954. In this way, the gas comprising hydrogen can be transferred between the humidifying cup 4, the condensation filter device 5, the filter 60, the nebulizer 7, the hydrogen water cup 8, the automatic diversion device 90 and the valve component 95 by the integrated channel device 3. In one embodiment, the humidifying cup 4, the condensation filter device 5, the nebulizer 7, the automatic diversion device 90 and the valve component 95 can be directly coupled to the lower cover 31.

As shown in FIG. 10 to FIG. 13, the humidifying chamber 40 has the humidifying space 400 for accommodating the supplementary water and includes a fining device43. The fining device43 includes a fining communication column 430, a coupling element 431 and a fining base 432. Wherein, the fining communication column 430 is coupled to the gas delivery export 954, and the fining communication column 430 has a first fining channel. The fining base 432 is coupled to the coupling element 431, and the coupling element 431 is coupled to one end of the fining communication column 430 away from the gas delivery export 954. The fining base 432 is immersed in the replenishing water. The fining base 432 further includes a fining cover 4320 and a fining body 4322, and the fining cover 4320 and the fining body 4322 are embedded with each other to form a second fining channel. The first fining channel and the second fining channel are connected with each other. Wherein, the fining cover 4320 has a plurality of fining holes 4321 for coupling with the humidifying space 400 and the second fining channel. In this way, the gas comprising hydrogen enters from the gas delivery export 954, then flows through the first fining channel, the second fining channel, the fining holes 4321, and finally enters into the humidifying chamber 40 and be injected with the replenishing water to humidify the gas comprising hydrogen. In one embodiment, the fining holes 4321 are centered at the intersection of the first fining channel and the second fining channel. In order to evenly distribute the amount of the gas comprising hydrogen output by each fining holes 4321, to closer to the center, the smaller the diameter of the fining holes 4321 became; the further away from the center, the larger the diameter of the fining holes 4321 became. In another one embodiment, the fining holes 4321 are centered at the intersection of the first fining channel and the second fining channel. Wherein, the diameter of the fining holes 4321, which are closer to the center, are inconsistent with the diameter of the fining holes 4321, which are far away from the center. For example, the diameter of the fining holes 4321 are uniform but gradually changes, so as to change the gas flow rate of the gas comprising hydrogen while passing through the second fining channel and the gas comprising hydrogen will not be output to the humidifying chamber 40 through some specific area of the second fining channel.

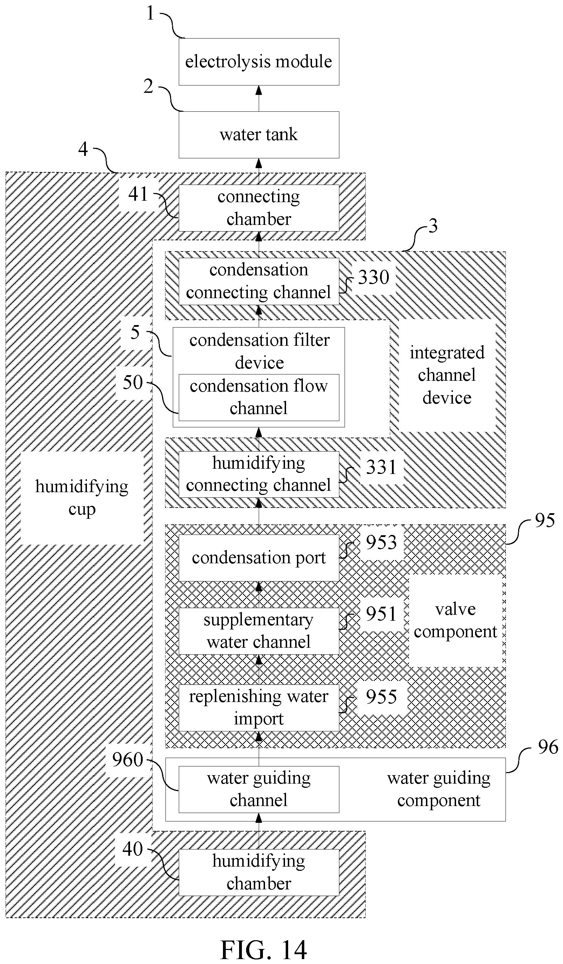

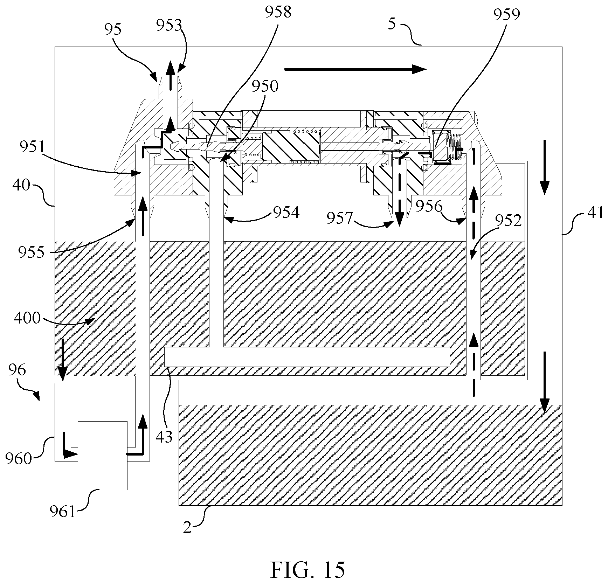

Regarding the water flow path, please refer to FIG. 14 and FIG. 15. FIG. 14 is a functional block diagram illustrating the valve component 95 of the hydrogen generator E with self-sterilization function according to another embodiment of the present invention. FIG. 15 is a schematic diagram of the valve component 95 of the hydrogen generator E of FIG. 14 under normal operating condition. As shown in FIG. 14 and FIG. 15, the valve component 95 further includes a supplementary water channel 951 coupled to the humidifying chamber 40 of the humidifying cup 4 and the condensation flow channel 50 of the condensation filter device 5 for transferring the replenishing water. Furthermore, the valve component 95 has the condensation port 953 and the replenishing water import 955 coupled to the supplementary water channel 951. The condensation port 953 is at least coupled to the condensation filter device 5. The replenishing water import 955 is at least coupled to the humidifying cup 4 for receiving the replenishing water from the humidifying cup 4 and outputting the replenishing water from the condensation port 953. The replenishing water flows through the condensation flow channel 50 of the condensation filter device 5 and enters into the water tank 2. When the replenishing water flows through the condensation filter device 5, the electrolytes filtered by the condensation filter device 5 will also be flushed back to the water tank 2. Through the above skill, on the one hand, the service life of the condensation filter device 5 can be extended, and on the other hand, can reduce the consumption of the electrolytes and increase the service life of the hydrogen generator E. Wherein, the timing of the replenishing water flows back can be performed when the electrolysis module 1 stops electrolyzing.

Wherein, in order to keep the gas flow path and the liquid flow path unobstructed and prevent the gas flow path and the liquid flow path from interfering with each other, the valve component 95 further includes a first valve element 958. Please refer to FIG. 11 and FIG. 15. As shown in FIG. 11 and FIG. 15, the first valve element 958 can selectively block the gas delivery channel 950, so that the condensation port 953 does not connect to the gas delivery export 954, and then the first valve element 958 connects the supplementary water channel 951, so that the condensation port 953 connects the replenishing water import 955, or the first valve element 958 can selectively block the supplementary water channel 951, so that the condensation port 953 v the replenishing water import 955, and then the first valve element 958 connects the gas delivery channel 950, so that the condensation port 953 is connected to the gas delivery export 954.

Selectively, the valve component 95 further includes an exhaust flow channel 952 and a second valve element 959. The exhaust flow channel 952 is coupled with the humidifying chamber 40 and the water tank 2. Furthermore, the valve component 95 has an exhaust import 956 and an exhaust export 957 connected with the exhaust flow channel 952. The exhaust import 956 is coupled with the exhaust flow channel 952 and the water tank 2. The exhaust export 957 is coupled with the exhaust flow channel 952 and the humidifying chamber 40. Wherein, after the replenishing water enters into the water tank 2, the gas comprising hydrogen in the water tank 2 can enter into the humidifying chamber 40 through the exhaust flow channel 952. The second valve element 959 is coupled to the exhaust flow channel 952 and configured to selectively connect the exhaust flow channel 952 to connect the humidifying chamber 40 and the water tank 2.

In one present embodiment, the first valve element 958 and the second valve element 959 are interconnected. When the first valve element 958 blocks the gas delivery channel 950 and then connects the supplementary water channel 951, the second valve element 959 connects the exhaust flow channel 952. When the first valve element 958 connects the gas delivery channel 950 and then blocks the supplementary water channel 951, the second valve element 959 blocks the exhaust flow channel 952. In this way, the gas comprising hydrogen in the humidifying chamber 40 will not enter into the water tank 2 through the exhaust flow channel 952 while the gas comprising hydrogen is continuously generated, so as to ensure the correct flow direction of the gas comprising hydrogen. In practical application, the first valve element 958 and the second valve element 959 can be actuated and controlled by solenoid valve.

In order to promote the replenishing water in the humidifying chamber 40 be transferred well to the condensation flow channel 50 above the humidifying chamber 40, the hydrogen generator E of the present invention further includes the water guiding component 96. As shown in FIG. 14 and FIG. 15, the water guiding component 96 includes a water guiding channel 960 and a pump 961. The water guiding component 96 is coupled with the humidifying chamber 40 and the supplementary water channel 951. The pump 961 is coupled to the water guiding channel 960 and is configured to drive the replenishing water to flow from the humidifying chamber 40 through the water guiding channel 960, the supplementary water channel 951, and the condensation flow channel 50 to arrive the water tank 2. That is, the pump 961 can drive the replenishing water in the humidifying chamber 40 to the condensation filter device 5 to flush back the electrolytes, and finally enter into the water tank 2 and/or the electrolysis module 1.

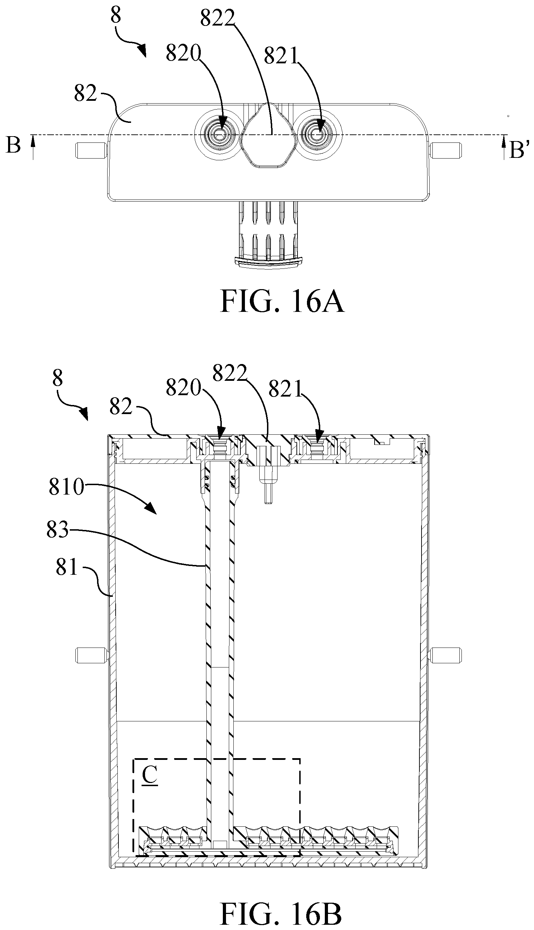

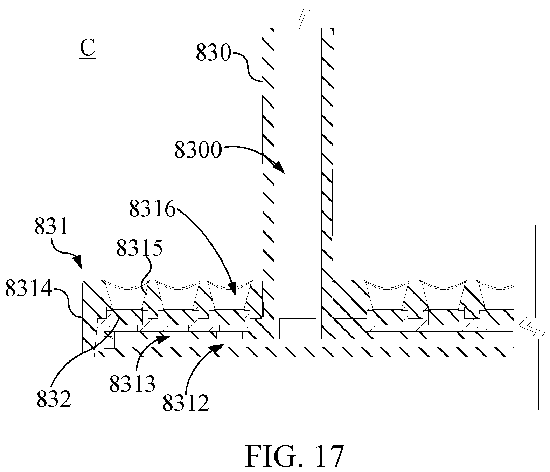

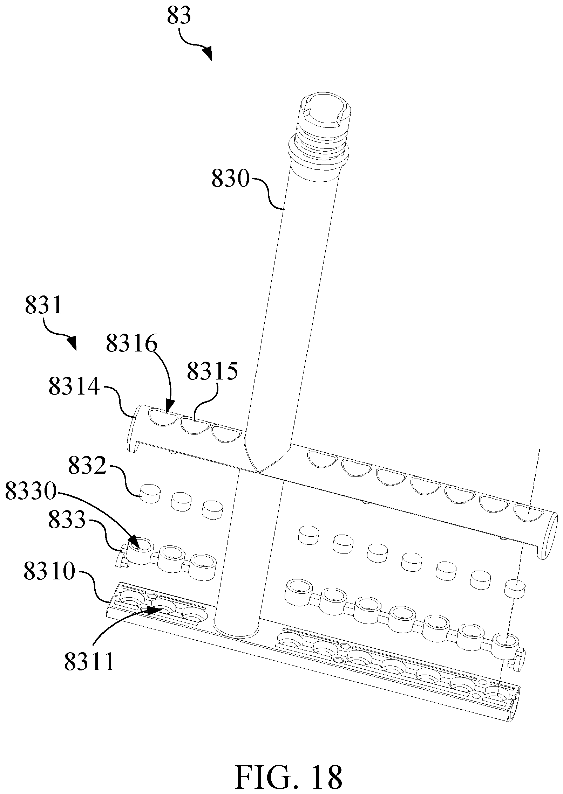

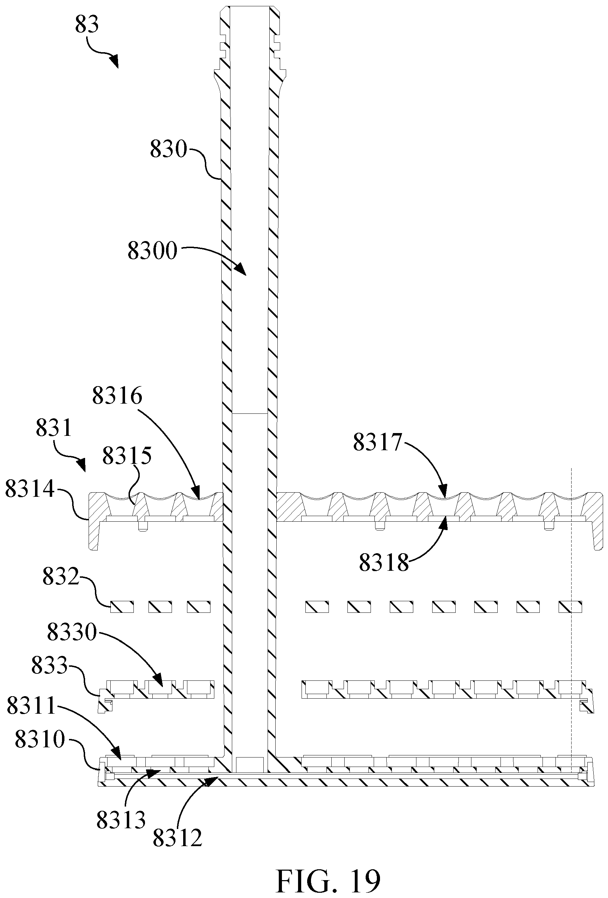

Detailed description for hydrogen water cup 8, please refer to FIG. 16A is a sectional diagram illustrating the section B-B' of the hydrogen water cup of FIG. 2. FIG. 16B is a sectional diagram along the section B-B' of FIG. 16A. FIG. 17 is a partially enlarged schematic diagram of the circled area C of FIG. 16B. FIG. 18 is an exploded diagram illustrating the gas injection component 83 of FIG. 16B. FIG. 19 is a sectional diagram illustrating the gas injection component 83 of FIG. 18. As shown in FIG. 16A and FIG. 16B, the hydrogen water cup 8 of the present invention includes a cup body 81, a cover 82 and a gas injection component 83. The cup body 81 has an accommodation space 810 for accommodating liquid or drinking water. The cover 82 is coupled to the cup body 81, and an air inlet 820 and an air outlet 821 are arranged on the cover 82. The gas injection component 83 is arranged in the accommodation space 810 and coupled to the air inlet 820 to be injected the gas comprising hydrogen into liquid or drinking water to form the liquid comprising hydrogen or the water comprising hydrogen. The cover 82 also includes a water inlet and outlet (not shown in the FIG. 16B) and a water outlet cover 822. The water inlet and outlet can replenish liquid into the hydrogen water cup 8 and output the gas comprising hydrogen. The water outlet cover 822 can cover the water inlet and outlet.

As shown in FIG. 16A to FIG. 19, the gas injection component 83 includes a gas injection column 830 and a gas injection base 831. The gas injection column 830 is couple to the air inlet 820 and has a first gas injection channel 8300. The gas injection base 831 can be arranged and immersed in the drinking water, and the gas injection base 831 further includes a gas injection base body 8310 and a gas injection cover 8314. The gas injection base body 8310 is couple to gas injection column 830 which has a second gas injection channel 8312 and a plurality of gas injection holes 8313. Wherein, the second gas injection channel 8312 is couple to the first gas injection channel 8300, and the plurality of gas injection holes 8313 are couple to the second gas injection channel 8312 (shown in FIG. 17). The gas injection cover 8314 is embedded into the gas injection base body 8310 and the gas injection cover 8314 has a micro-bubble gas outlet structure 8315 to change the gas comprising hydrogen after fined to form a plurality of micro-bubbles in the drinking water. Wherein, the micro-bubble gas outlet structure 8315 has a plurality of micro air outlet channels 8316 corresponding to the gas injection holes 8313, and the micro air outlet channels 8316 are coupled to the second gas injection channel 8312 through the gas injection holes 8313. When the gas comprising hydrogen enters into the hydrogen water cup 8 from the air inlet 820, the gas comprising hydrogen flows through the first gas injection channel 8300, the second gas injection channel 8312 and the micro air outlet channel 8316 in sequence, and then forms a plurality of micro-bubbles in the drinking water through the micro-bubble gas outlet structure 8315.

As shown in FIG. 18 and FIG. 19, the gas injection component 83 further includes a plurality of micro filter elements 832, respectively coupled to each micro air outlet channel 8316. The micro filter elements 832 are configured to filter the gas comprising hydrogen flowing through the air outlet channel 8316 to ensure the quality of the gas comprising hydrogen injected into the drinking water is safe. In practical application, the micro filter elements 832 can be an activated carbon filter element, a drinking water filter element, etc., but is not limited thereto. In addition, the micro filter elements 832 can further refine the gas comprising hydrogen into micro-bubbles to increase the contact area with the drinking water, so as to increase the concentration of the gas comprising hydrogen in the drinking water.

As shown in FIG. 17 and FIG. 19, the air outlet channel 8316 is a hollow truncated cone structure which has an upper hole 8317 and a lower hole 8318. Wherein, in one present embodiment, the area of the upper hole 8317 is larger than the area of the lower hole 8318. The lower hole 8318 is located between the second gas injection channel 8312 and the micro air outlet channel 8316, and the upper hole 8317 is located between the micro air outlet channel 8316 and the accommodation space 810. Since the hollow truncated cone structure of the gas injection component 83 of the present invention, the micro-bubble can be dispersed more and be injected into the drink water. On the contrary, if the area of the upper hole 8317 is larger than the area of the lower hole 8318, the gas comprising hydrogen will accumulate and form a larger bubble state, thereby reducing the contact area of gas comprising hydrogen after injected into the drink water.

In order to evenly distribute the amount of the gas comprising hydrogen flowing out from each micro air outlet channel 8316, the second gas injection channel 8312 of the gas injection component 83 of the present invention gradually becomes larger from the joint between the first gas injection channel 8300 to the both ends of the gas injection base body 8310, so as to improve the output efficiency of the gas comprising hydrogen in micro-bubble state and the uniformity of dispersion in the drinking water. In other words, two ends of the second gas injection channel 8312 can be designed wider than the middle of the second gas injection channel 8312, which can let all micro air outlet channels 8316 be fully utilized and increase the flow rate of the gas comprising hydrogen while the gas comprising hydrogen flowing through the two ends of the second gas injection channel 8312, so as to prevent most of the gas comprising hydrogen from being injected into the water from the joint between the first gas injection channel 8300 and the second gas injection channel 8312.

The gas injection component 83 further includes a fixing element 833. The fixing element 833 has a plurality of fixing holes 8330 for accommodating and fixing the micro filter elements 832. The surface of the gas injection base body 8310 which is facing the gas injection cover 8314 has a groove 8311 for accommodating the fixing element 833. In one present embodiment, the fining device 43 is also equipped with the design such as the gas injection component 83. In other words, the fining holes 4321 of the fining device 43 can also be designed as the micro-bubble gas outlet structure 8315 to improve the fining effect.

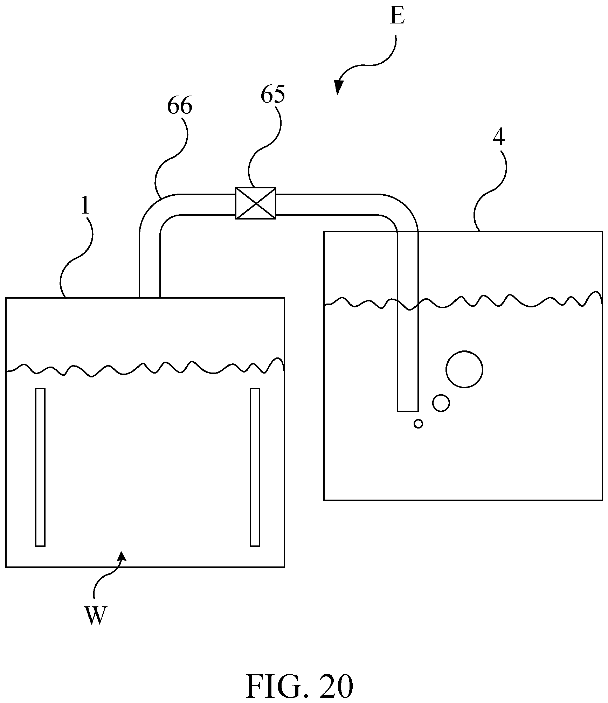

Please refer to FIG. 20. FIG. 20 is a schematic diagram illustrating the hydrogen generator E with self-sterilization function according to another embodiment of the present invention. As shown in FIG. 20, the hydrogen generator E with self-sterilization function includes an electrolysis module 1 for accommodating electrolytic water W. The electrolysis module 1 can electrolyze the electrolytic water W located therein to generate and output the gas comprising hydrogen. It should be noted that in order to show simply in FIG. 20, the water tank is hidden. In this present embodiment, the hydrogen generator E still includes the water tank 2 as the previous embodiment, and the electrolysis module 1 is arranged in the water tank 2 to receive the electrolytic water W from the water tank 2. The electrolytic water W in the electrolysis module 1 includes electrolytes with 0.1% weight percentage concentration or larger than 0.1% volume percentage concentration (for example, between 0.5% -15%). Therefore, the electrolytes in the electrolysis module 1 and the water tank (not shown in FIG. 20) can form the strong alkaline environment with pH value is larger than 12. Furthermore, if the electrolytic water W includes electrolytes with 1% weight percentage concentration or larger than 1% volume percentage concentration or a greater concentration, at this time, the strong alkaline environment in the electrolysis module 1 and the water tank with pH value is larger than 13.4.

It is known from experiments that the electrolytic water in the electrolysis module 1 and the water tank of the present invention includes electrolytes over than 0.1% concentration, at this time, the pH value in the internal environment of the electrolysis module 1 and the water tank can reach above 12. When the electrolytic water includes electrolytes with 1% concentration, the pH value in the electrolytic water can reach to 13.4. When the electrolytic water includes electrolytes with 2% concentration, the pH value in the electrolytic water can reach to 13.7. When the electrolytic water includes electrolytes with 3% concentration, the pH value in the electrolytic water can reach to 13.88. When the electrolytic water includes electrolytes with 4% concentration or over than 4%, the pH value in the electrolytic water can reach to 14. The experimental result shows that there are no germs can survive under the strong alkaline environment with pH value is between 12-14. However, too much concentration of the electrolytes will easily generate alkali mist, which will easily harm the human respiratory system if the alkali mist will not be filtered. On the contrary, the electrolysis efficiency decreases when the concentration of the electrolytes in the electrolytic water is less than 0.1%. On the other hand, the experimental result shows that when the electrolytic water includes electrolytes with 6% concentration, the hydrogen generator will generate the minimum of the noise. Therefore, according to the above factors such as sterilization, electrolysis efficiency, alkali mist and noise, the suitable electrolyte concentration range of the hydrogen generator of the present invention is between 0.5%-15% which can form a strong alkaline environment with pH value in the range between 12-14 in the electrolysis module for effective sterilization. It is known from experiments that if the electrolyte concentration in the electrolyzed water is between 1%-3%, the above-mentioned strong alkaline environment with pH value is between 13-13.9.

The experimental result shows that the strong alkaline environment in the water tank 2 and the electrolysis module 1 is enough to prevent all Legionella pneumophila from surviving. In addition, Mycobacterium tuberculosis is a representative of alkali-resistant bacteria. When the electrolytic water W includes electrolytes with 0.1% concentration, the pH value in the electrolytic water W is larger than 12 to inactivate Mycobacterium tuberculosis. Furthermore, when the electrolytic water W includes electrolytes with 1% concentration, the pH value in the electrolytic water W is larger than 13.4 or even close to 14, at this time, Mycobacterium tuberculosis or other alkali-resistant bacteria cannot survive.

Next, since the black variant spores of Bacillus subtilis is able to highly resistant to heat, ultraviolet light, ionizing radiation and certain chemicals, the black variant spores of Bacillus subtilis is considered to be the most difficult to be eliminated in bacterial species. Therefore, in some countries such as The United States, the United Kingdom, Japan, the European Union or other countries have added the black variant spores of Bacillus subtilis in food testing and medical testing as a quality control standard testing strain. Moreover, the black variant spores of Bacillus subtilis has been used internationally as an indicator bacterium for evaluation tests of bactericidal effects of chemical disinfectants, dry heat, and ethylene oxide. In addition, the black variant spores of Bacillus subtilis is a representative strain for high-level disinfection methods against disinfectants or disinfection equipment, the Chinese Ministry of Health has also added the black variant spores of Bacillus subtilis as a standard testing strain in the "Technical Specifications for Disinfection".

The hydrogen generator E with self-sterilization function of the present invention also has the ability to eliminate the black variant spores of Bacillus subtilis. As mentioned previously, the pH value in the alkaline environment in the electrolysis module and the water tank of the hydrogen generator is over than 12, and can even reach 13.8 or above. The experimental result shows that the black variant spores of Bacillus subtilis cannot survive in the electrolytic water of the water tank and the electrolysis module which include electrolytes over than 0.1% concentration and the pH value in the alkaline environment is over than 12. In addition, as mentioned above, the gas comprising hydrogen generated by the electrolysis module also contains moisture from the electrolytic water with a high pH value, at this time, the gas comprising hydrogen can also sterilize or disinfect the gas flow channel.

In another one embodiment, the humidifying cup is connected to the gas flow path which is formed between the water tank, the electrolysis module, the hydrogen water cup, the automatic diversion device, the condensation filter device and other units through the integrated channel device. The humidifying cup can also transfer the replenishing water through the same path. Namely, the gas flow path and the liquid flow path use the same path as mentioned, but the flow direction of the gas comprising hydrogen and the replenishing water are opposite. In an experiment, the hydrogen generator E of the present invention is input a large amount of the black variant spores of Bacillus subtilis into the humidifying cup from the outside. The black variant spores of Bacillus subtilis will enter the above-mentioned units, especially the water tank, which can simulate the situation where the electrolytic water in the water tank is contaminated by pathogens. Next, turn on the hydrogen generator and continue operating, measure the microbial load of the gas output from the gas output port of the hydrogen generator.

In the above experiment, after the hydrogen generator was continued to operate for 30 minutes to 1 hour, there is no microorganism detected at the gas output port. Furthermore, after operating for 23.5 hours to 24 hours, there is still no microorganism detected at the gas output port. In addition, after operating for24 hours, the total number of bacteria in the water tank was reduced by more than 99%. Next, the above experimental results were converted into a log killing value of microorganisms by the electrolysis module, and the value is over than 6.46, which is far exceeding the requirement of a log killing value of 5 for high-level disinfection in the "Technical Specifications for Disinfection". Through the above experiment, the hydrogen generator of the present invention can eliminate the most difficult-to-eliminate bacteria in the world while maintaining the purity of the output gas, and has excellent disinfection and sterilization effects.

In the above embodiments, although the higher the electrolyte concentration in the electrolytic water, the higher pH value is beneficial to sterilization. However, too high electrolyte concentration may reduce the efficiency of electrolyzing the gas comprising hydrogen. Therefore, in practice, the electrolyte concentration in the electrolytic water can be maintained between 0.5% and 15%, and further between 1% and 3%, which can achieve excellent disinfection and sterilization effects in the alkaline environment with high pH value in the electrolysis module and the water tank, and can make the electrolysis module maintain high production efficiency.

Please refer to FIG. 20 again. In this embodiment, the gas comprising hydrogen, electrolyzed and generated from the electrolytic water W by the electrolysis module 1, will enter into the gas flow channel 66 and be transferred to other units or modules in the hydrogen generator E, such as the humidifying cup 4. It should be noted that the gas flow channel 66 in FIG. 20 is a pipeline connecting the electrolysis module 1 and the humidifying cup 4. In practice, any path that the gas comprising hydrogen can flow through in the hydrogen generator E is part of the gas flow channel 66, such as the gas flow channel formed between the integrated channel device, the electrolysis module, the hydrogen water cup and the automatic diversion device in the aforementioned embodiment. The hydrogen generator E includes a filter cotton 65 arranged in the gas flow channel 66. Therefore, when the gas comprising hydrogen flows through the filter cotton 65, the germs will be filtered by the filter cotton 65. In addition, in practice, the filtering and sterilizing bottle, the ozone generator and the ultraviolet light generator of the aforementioned embodiment can also be applied in this embodiment, and can be combined with filter cotton to obtain better sterilization and disinfection effects.

Compared with the prior art, the hydrogen generator E of the present invention includes the automatic diversion device 90, which can selectively allow the gas comprising hydrogen to flow through the hydrogen water cup 8 according to the diversion signal, and also control the nebulizer, so as to reduce the problem caused by the low-frequency sound when the gas comprising hydrogen is injected into the hydrogen water cup 8 and the nebulizer 7 generates the atomized gas. In addition, the hydrogen water cup 8 of the hydrogen generator E of the present invention has the micro-bubble gas outlet structure 8315, which has the micro air outlet channel 8316 with a hollow truncated cone structure. The micro air outlet channel 8316 can make the fine gas comprising hydrogen form micro-bubbles in the drinking water and inject it into the drinking water to form the water comprising hydrogen, so as to increase the contact area of the gas comprising hydrogen in the drinking water and enhance the concentration of the gas comprising hydrogen in the drinking water.

Furthermore, the integrated channel device includes many channels that can be directly coupled to the nebulizer, the condensation filter device, the hydrogen water cup, etc.; and the integrated channel device is vertically stacked above the humidifying cup, and the humidifying cup is vertically stacked above the water tank. The condensation filter device in the integrated channel device can receive the gas comprising hydrogen from the water tank through the connecting room of the hydrogen water cup. Therefore, the communication between the components of the hydrogen generator E does not require to add additional pipelines (such as general air pipes or water pipes), so as to reduce the risk of air and water leaks.

The hydrogen generator of the present invention can continuously perform self-disinfection and self-sterilization function without being disassembled due to the alkaline electrolysis environment, the temperature environment that is not conducive to the survival of germs or germs, and different devices such as the ozone generators, the ultraviolet light generators, and the filter sterilization tanks installed inside or outside the hydrogen generator. Moreover, the hydrogen generator of the present invention can not only provide users with germ-free, the pure gas comprising hydrogen or the health caring gas, but also significantly reduce the maintenance cost of the hydrogen generator.

With the examples and explanations mentioned above, the features and spirits of the invention are hopefully well described. More importantly, the present invention is not limited to the embodiment described herein. Those skilled in the art will readily observe that numerous modifications and alterations of the device may be made while retaining the teachings of the invention. Accordingly, the above disclosure should be construed as limited only by the metes and bounds of the appended claims.

## Claims

1. A hydrogen generator with self-sterilization function, comprising:
an electrolysis module configured to electrolyze an electrolytic water to generate a gas comprising hydrogen;
a hydrogen water cup configured to accommodate a liquid, the hydrogen water cup being configured to input the gas comprising hydrogen into the liquid to generate a liquid comprising hydrogen;
an integrated channel device stacked above the electrolysis module, the integrated channel device having a gas input channel, a gas output channel, and a gas flow channel, wherein the gas input channel is configured to receive the gas comprising hydrogen, and the gas output channel is configured to output the gas comprising hydrogen; and
an automatic diversion device selectively connected with the gas input channel, the gas output channel and the gas flow channel to input the gas comprising hydrogen into the hydrogen water cup and to output the gas comprising hydrogen through the gas output channel; the automatic diversion device selectively connected with the gas input channel, the gas flow channel and the gas output channel to output the gas comprising hydrogen through the gas flow channel and the gas output channel;
wherein, the pH value of the electrolytic water in the electrolysis module is in a range between 12-14 to generate a strong alkaline environment for sterilization.

2. The hydrogen generator with self-sterilization function of claim 1, wherein the internal temperature of the electrolysis module is in a range between 50°C to 80°C while the electrolysis module performs electrolysis.

3. The hydrogen generator with self-sterilization function of claim 1, wherein the pH value of the electrolytic water in the electrolysis module is in a range between 13-13.9.

4. The hydrogen generator with self-sterilization function of claim 1, further comprising a nebulizer coupled to the gas output channel to receive the gas comprising hydrogen, wherein the nebulizer selectively generates an atomized gas to be mixed with the gas comprising hydrogen to form a healthy gas.

5. The hydrogen generator with self-sterilization function of claim 4, wherein the nebulizer generates the atomized gas when the gas comprising hydrogen is inputted into the hydrogen water cup and then outputted from the gas output channel; the nebulizer stops generating the atomized gas when the gas comprising hydrogen is outputted by the gas output channel through the gas flow channel.

6. The hydrogen generator with self-sterilization function of claim 1, further comprising a frame for the hydrogen water cup to be embedded in to couple the hydrogen water cup to the integrated channel device, wherein the electrolysis module stops operating when the hydrogen water cup is detached from the frame and not coupled to the integrated channel device.

7. The hydrogen generator with self-sterilization function of claim 1, further comprising a condensation filter device coupled to the integrated channel device to condense and filter the gas comprising hydrogen; wherein the integrated channel device comprises a lower cover, the lower cover is an integrally formed structure and directly coupled to the automatic diversion device and the condensation filter device, the lower cover has a space configured to accommodate the condensation filter device and a movable flip-up structure, configured to movably embed the condensation filter device into the integrated channel device; wherein the gas comprising hydrogen is transferred between the hydrogen water cup, the automatic diversion device and the condensation filter device by the integrated channel device.

8. The hydrogen generator with self-sterilization function of claim 7, further comprising:
a water tank stacked below the integrated channel device and coupled to the electrolysis module, the water tank being configured to accommodate the electrolytic water and receive the gas comprising hydrogen from the electrolysis module;
a humidifying cup stacked above the water tank, the humidifying cup having a filter chamber and a humidifying chamber for accommodating a replenishing water; and
a filter accommodated in the filter chamber to filter the gas comprising hydrogen flowing through the filter chamber;
wherein, the gas comprising hydrogen is transferred between the hydrogen water cup, the automatic diversion device, the condensation filter device, the humidifying cup and the filter by the integrated channel device; wherein, the automatic diversion device, the condensation filter device and the humidifying cup are directly coupled to the lower cover.

9. The hydrogen generator with self-sterilization function of claim 8, wherein the condensation filter device comprises a condensation flow channel, the lower cover comprises a condensation connecting channel coupled to the condensation flow channel, the humidifying cup comprises a connecting chamber coupling the water tank and the condensation connecting channel, wherein the humidifying chamber, the connecting chamber and the filter chamber of the humidifying cup are separated from each other.

10. The hydrogen generator with self-sterilization function of claim 9, further comprising an electrolyte filtering module configured in the connecting chamber of the humidifying cup, the electrolyte filtering module having a continuous upward slope channel to receive and filter the gas comprising hydrogen from the water tank.

11. The hydrogen generator with self-sterilization function of claim 1, further comprising an ozone generator, wherein the ozone generator is coupled to a gas flow channel which is formed by the integrated channel device, the electrolysis module, the hydrogen water cup and the automatic diversion device for the gas comprising hydrogen to flow therein, wherein the ozone generator is configured to generate an ozone gas into the gas flow channel to sterilize the gas flow channel when the electrolysis module stops electrolyzing.

12. The hydrogen generator with self-sterilization function of claim 1, wherein the integrated channel device, the electrolysis module, the hydrogen water cup and the automatic diversion device form a gas flow channel for the gas comprising hydrogen to flow therein, and the gas flow channel is configured to be connected to an ozone generator out of the hydrogen generator, so as to receive an ozone gas generated by the ozone generator from the outside to sterilize the gas flow channel when the electrolysis module stops electrolyzing.

13. The hydrogen generator with self-sterilization function of claim 1, further comprising an ultraviolet light generator coupled to a gas flow channel formed by the integrated channel device, the electrolysis module, the hydrogen water cup and the automatic diversion device, wherein the ultraviolet light generator is configured to generate an ultraviolet light onto the gas flow channel to sterilize the gas in the gas flow channel.

14. A hydrogen generator with self-sterilization function, comprising:
an electrolysis module configured to electrolyze an electrolytic water to generate a gas comprising hydrogen;
a water tank configured to accommodate the electrolytic water and the electrolysis module, the water tank being configured to receive the gas comprising hydrogen from the electrolysis module;
a condensation filter device stacked above the water tank and configured to receive and filter the gas comprising hydrogen;
a humidifying cup stacked above the water tank, the humidifying cup being configured to accommodate a replenishing water, the humidifying cup being configured to receive and humidify the gas comprising hydrogen from the condensation filter device; and
an integrated channel device stacked above the water tank and respectively coupled to the condensation filter device and the humidifying cup;
wherein, the gas comprising hydrogen is transferred between the condensation filter device and the humidifying cup through the integrated channel device;
wherein, the pH value of the electrolytic water in the electrolysis module is in a range between 12-14 to generate a strong alkaline environment for sterilization.

15. The hydrogen generator with self-sterilization function of claim 14, further comprising a filtering and sterilizing bottle detachably coupled to a gas output port of the hydrogen generator for filtering germs in the gas comprising hydrogen outputted from the gas output port.

16. The hydrogen generator with self-sterilization function of claim 14, further comprising an ozone generator, wherein the hydrogen generator has a gas flow channel for the gas comprising hydrogen to flow therein, and the ozone generator is coupled to the gas flow channel, the ozone generator is configured to generate an ozone into the gas flow channel to sterilize the gas flow channel when the electrolysis module stops electrolyzing.

17. The hydrogen generator with self-sterilization function of claim 14, wherein the hydrogen generator further has a gas flow channel for the gas comprising hydrogen to flow therein, and the gas flow channel is connected to an ozone generator out of the hydrogen generator to receive an ozone gas generated by the ozone generator from the outside to sterilize the gas flow channel when the electrolysis module stops electrolyzing.

18. The hydrogen generator with self-sterilization function of claim 14, further comprising an ultraviolet light generator coupled to a gas flow channel of the hydrogen generator, wherein the ultraviolet light generator is configured to generate an ultraviolet light onto the gas flow channel to sterilize the gas in the gas flow channel.

19. The hydrogen generator with self-sterilization function of claim 14, wherein the pH value of the electrolytic water in the electrolysis module is in a range between 13-13.9.

20. A hydrogen generator with self-sterilization function, comprising:
an electrolysis module configured to electrolyze an electrolytic water to generate a gas comprising hydrogen; and
a water tank configured to accommodate the electrolytic water and coupled to the electrolysis module, the water tank being configured to provide the electrolytic water to the electrolysis module and receive the gas comprising hydrogen from the electrolysis module;
wherein, the electrolytic water in the electrolysis module comprises electrolytes with 0.5%-15% weight percentage concentration or volume percentage concentration, so as to form a strong alkaline environment with pH value in a range between 12-14 in the electrolysis module for sterilization.

21. The hydrogen generator with self-sterilization function of claim 20, further comprising a gas flow channel for receiving the gas comprising hydrogen to flow therein, the gas flow channel comprising a gas output port to output the gas comprising hydrogen, and the hydrogen generator further comprising a filtering and sterilizing bottle coupled to the gas output port for filtering germs in the gas comprising hydrogen output from the gas output port.

22. The hydrogen generator with self-sterilization function of claim 20, further comprising a gas flow channel for receiving the gas comprising hydrogen to flow therein, and an ozone generator coupled to the gas flow channel, wherein the ozone generator is configured to generate an ozone gas into the gas flow channel to sterilize the gas flow channel when the electrolysis module stops electrolyzing.

23. The hydrogen generator with self-sterilization function of claim 20, further comprising a gas flow channel for receiving the gas comprising hydrogen to flow therein, wherein the gas flow channel is configured to be connected to an ozone generator out of the hydrogen generator, so as to receive an ozone gas generated by the ozone generator from the outside to sterilize the gas flow channel.

24. The hydrogen generator with self-sterilization function of claim 20, further comprising a gas flow channel for receiving the gas comprising hydrogen to flow therein, the hydrogen generator further comprising an ultraviolet light generator coupled to the gas flow channel, wherein the ultraviolet light generator is configured to generate an ultraviolet light onto the gas flow channel to sterilize the gas in the gas flow channel.

25. The hydrogen generator with self-sterilization function of claim 20, wherein the pH value of the electrolytic water in the electrolysis module is in a range between 13-13.9.
